# EUROPÄISCHE PATENTANMELDUNG

(11) **EP 2 653 538 A1**
(43) Veröffentlichungstag der Anmeldung: **23.10.2013**
(21) Anmeldenummer: 12164933.9
(22) Anmeldetag: 20.04.2012
(51) Int. Cl.: C12N 9/00, C12P 13/06, C12P 7/24

(54) **NADP-abhängige Alanindehydrogenase**

(71) Anmelder: Evonik Industries AG, 45128 Essen (DE)
(72) Erfinder: Haas, Thomas, 48161 Münster (DE); Pfeffer, Jan, 45355 Essen (DE); Skerra, Arne, 85354 Freising (DE); Lerchner, Alexandra, 80992 München (DE); Jarasch, Alexander, 80796 München (DE)

(57) **Zusammenfassung**

Die vorliegende Erfindung betrifft ein Polypeptid umfassend die Aminosäuresequenz der Alanindehydrogenase aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist, ein Nukleinsäuremolekül kodierend für eine derartiges Polypeptid sowie ein Verfahren zur Herstellung von Alanin oder einer unter Verbrauch von Alanin entstehenden Verbindung umfassend den Schritt Umsetzen von Pyruvat mit Ammonium und NADPH zu Alanin durch Kontaktieren mit dem erfindungsgemäßen Polypeptid oder der erfindungsgemäßen Zelle.

SEQUENCE LISTING

<110>  Evonik Industries AG

<120>  NADP-abhängige Alanindehydrogenase

<130>  201200079

<160>  23

<170>  PatentIn version 3.5

<210>  1
<211>  378
<212>  PRT
<213>  Bacillus subtilis

<400>  1

Met Ile Ile Gly Val Pro Lys Glu Ile Lys Asn Asn Glu Asn Arg Val
1               5                   10                  15

Ala Leu Thr Pro Gly Gly Val Ser Gln Leu Ile Ser Asn Gly His Arg
            20                  25                  30

Val Leu Val Glu Thr Gly Ala Gly Leu Gly Ser Gly Phe Glu Asn Glu
        35                  40                  45

Ala Tyr Glu Ser Ala Gly Ala Glu Ile Ile Ala Asp Pro Lys Gln Val
    50                  55                  60

Trp Asp Ala Glu Met Val Met Lys Val Lys Glu Pro Leu Pro Glu Glu
65                  70                  75                  80

Tyr Val Tyr Phe Arg Lys Gly Leu Val Leu Phe Thr Tyr Leu His Leu
                85                  90                  95

Ala Ala Glu Pro Glu Leu Ala Gln Ala Leu Lys Asp Lys Gly Val Thr
            100                 105                 110

Ala Ile Ala Tyr Glu Thr Val Ser Glu Gly Arg Thr Leu Pro Leu Leu
        115                 120                 125

Thr Pro Met Ser Glu Val Ala Gly Arg Met Ala Ala Gln Ile Gly Ala
    130                 135                 140

Gln Phe Leu Glu Lys Pro Lys Gly Gly Lys Gly Ile Leu Leu Ala Gly
145                 150                 155                 160

Val Pro Gly Val Ser Arg Gly Lys Val Thr Ile Ile Gly Gly Gly Val
                165                 170                 175

Val Gly Thr Asn Ala Ala Lys Met Ala Val Gly Leu Gly Ala Asp Val
            180                 185                 190

Thr Ile Ile Ala Arg Asn Ala Asp Arg Leu Arg Gln Leu Asp Asp Ile
        195                 200                 205

Phe Gly His Gln Ile Lys Thr Leu Ile Ser Asn Pro Val Asn Ile Ala
    210                 215                 220

Asp Ala Val Ala Glu Ala Asp Leu Leu Ile Cys Ala Val Leu Ile Pro
225                 230                 235                 240

Gly Ala Lys Ala Pro Thr Leu Val Thr Glu Glu Met Val Lys Gln Met
                245                 250                 255

Lys Pro Gly Ser Val Ile Val Asp Val Ala Ile Asp Gln Gly Gly Ile
            260                 265                 270

Val Glu Thr Val Asp His Ile Thr Thr His Asp Gln Pro Thr Tyr Glu
        275                 280                 285

Lys His Gly Val Val His Tyr Ala Val Ala Asn Met Pro Gly Ala Val
    290                 295                 300

Pro Arg Thr Ser Thr Ile Ala Leu Thr Asn Val Thr Val Pro Tyr Ala
305                 310                 315                 320

Leu Gln Ile Ala Asn Lys Gly Ala Val Lys Ala Leu Ala Asp Asn Thr
                325                 330                 335

Ala Leu Arg Ala Gly Leu Asn Thr Ala Asn Gly His Val Thr Tyr Glu
            340                 345                 350

Ala Val Ala Arg Asp Leu Gly Tyr Glu Tyr Val Pro Ala Glu Lys Ala
        355                 360                 365

Leu Gln Asp Glu Ser Ser Val Ala Gly Ala
    370                 375

<210>  2
<211>  1137
<212>  DNA
<213>  Bacillus subtilis

<400>  2
atgatcatag gggttcctaa agagataaaa aacaatgaaa accgtgtcgc attaacaccc      60

gggggcgttt ctcagctcat ttcaaacggc caccgggtgc tggttgaaac aggcgcgggc     120

cttggaagcg gatttgaaaa tgaagcctat gagtcagcag gagcggaaat cattgctgat     180

ccgaagcagg tctgggacgc cgaaatggtc atgaaagtaa aagaaccgct gccggaagaa     240

tatgtttatt ttcgcaaagg acttgtgctg tttacgtacc ttcatttagc agctgagcct     300

gagcttgcac aggccttgaa ggataaagga gtaactgcca tcgcatatga aacggtcagt 360

gaaggccgga cattgcctct tctgacgcca atgtcagagg ttgcgggcag aatggcagcg 420

caaatcggcg ctcaattctt agaaaagcct aaaggcggaa aaggcattct gcttgccggg 480

gtgcctggcg tttcccgcgg aaaagtaaca attatcggag gaggcgttgt cgggacaaac 540

gcggcgaaaa tggctgtcgg cctcggtgca gatgtgacga tcattgcccg taacgcagac 600

cgcttgcgcc agcttgatga catcttcggc catcagatta aaacgttaat ttctaatccg 660

gtcaatattg ctgatgctgt ggcggaagcg gatctcctca tttgcgcggt attaattccg 720

ggtgctaaag ctccgactct tgtcactgag gaaatggtaa aacaaatgaa acccggttca 780

gttattgttg atgtagcgat cgaccaaggc ggcatcgtcg aaactgtcga ccatatcaca 840

acacatgatc agccaacata tgaaaaacac ggggttgtgc attatgctgt agcgaacatg 900

ccaggcgcag tccctcgtac atcaacaatc gccctgacta acgttactgt tccatacgcg 960

ctgcaaatcg cgaacaaagg ggcagtaaaa gcgctcgcag acaatacggc actgagagcg 1020

ggtttaaaca ccgcaaacgg acacgtgacc tatgaagctg tagcaagaga tctaggctat 1080

gagtatgttc ctgccgagaa agctttacag gatgaatcat ctgtggcggg tgcttaa 1137

<210> 3
<211> 353
<212> PRT
<213> E. coli

<400> 3

Met Leu Tyr Thr Ser Gln Thr Thr Pro Glu Lys Asp Gln Lys Met Ser
1               5                   10                  15

Met Ile Lys Ser Tyr Ala Ala Lys Glu Ala Gly Gly Glu Leu Glu Val
            20                  25                  30

Tyr Glu Tyr Asp Pro Gly Glu Leu Arg Pro Gln Asp Val Glu Val Gln
        35                  40                  45

Val Asp Tyr Cys Gly Ile Cys His Ser Asp Leu Ser Met Ile Asp Asn
    50                  55                  60

Glu Trp Gly Phe Ser Gln Tyr Pro Leu Val Ala Gly His Glu Val Ile
65                  70                  75                  80

Gly Arg Val Val Ala Leu Gly Ser Ala Ala Gln Asp Lys Gly Leu Gln
                85                  90                  95

Val Gly Gln Arg Val Gly Ile Gly Trp Thr Ala Arg Ser Cys Gly His
            100                 105                 110

Cys Asp Ala Cys Ile Ser Gly Asn Gln Ile Asn Cys Glu Gln Gly Ala
        115                 120                 125

Val Pro Thr Ile Met Asn Arg Gly Gly Phe Ala Glu Lys Leu Arg Ala
    130                 135                 140

Asp Trp Gln Trp Val Ile Pro Leu Pro Glu Asn Ile Asp Ile Glu Ser
145                 150                 155                 160

Ala Gly Pro Leu Leu Cys Gly Gly Ile Thr Val Phe Lys Pro Leu Leu
                165                 170                 175

Met His His Ile Thr Ala Thr Ser Arg Val Gly Val Ile Gly Ile Gly
            180                 185                 190

Gly Leu Gly His Ile Ala Ile Lys Leu Leu His Ala Met Gly Cys Glu
        195                 200                 205

Val Thr Ala Phe Ser Ser Asn Pro Ala Lys Glu Gln Glu Val Leu Ala
    210                 215                 220

Met Gly Ala Asp Lys Val Val Asn Ser Arg Asp Pro Gln Ala Leu Lys
225                 230                 235                 240

Ala Leu Ala Gly Gln Phe Asp Leu Ile Ile Asn Thr Val Asn Val Ser
                245                 250                 255

Leu Asp Trp Gln Pro Tyr Phe Glu Ala Leu Thr Tyr Gly Gly Asn Phe
            260                 265                 270

His Thr Val Gly Ala Val Leu Thr Pro Leu Ser Val Pro Ala Phe Thr
        275                 280                 285

Leu Ile Ala Gly Asp Arg Ser Val Ser Gly Ser Ala Thr Gly Thr Pro
    290                 295                 300

Tyr Glu Leu Arg Lys Leu Met Arg Phe Ala Ala Arg Ser Lys Val Ala
305                 310                 315                 320

Pro Thr Thr Glu Leu Phe Pro Met Ser Lys Ile Asn Asp Ala Ile Gln
                325                 330                 335

His Val Arg Asp Gly Lys Ala Arg Tyr Arg Val Val Leu Lys Ala Asp
            340                 345                 350

Phe

<210> 4

<211>  349
<212>  PRT
<213>  E. coli

<400>  4

Met Lys Ile Lys Ala Val Gly Ala Tyr Ser Ala Lys Gln Pro Leu Glu
1               5                   10                  15

Pro Met Asp Ile Thr Arg Arg Glu Pro Gly Pro Asn Asp Val Lys Ile
            20                  25                  30

Glu Ile Ala Tyr Cys Gly Val Cys His Ser Asp Leu His Gln Val Arg
        35                  40                  45

Ser Glu Trp Ala Gly Thr Val Tyr Pro Cys Val Pro Gly His Glu Ile
    50                  55                  60

Val Gly Arg Val Val Ala Val Gly Asp Gln Val Glu Lys Tyr Ala Pro
65                  70                  75                  80

Gly Asp Leu Val Gly Val Gly Cys Ile Val Asp Ser Cys Lys His Cys
                85                  90                  95

Glu Glu Cys Glu Asp Gly Leu Glu Asn Tyr Cys Asp His Met Thr Gly
            100                 105                 110

Thr Tyr Asn Ser Pro Thr Pro Asp Glu Pro Gly His Thr Leu Gly Gly
        115                 120                 125

Tyr Ser Gln Gln Ile Val Val His Glu Arg Tyr Val Leu Arg Ile Arg
    130                 135                 140

His Pro Gln Glu Gln Leu Ala Ala Val Ala Pro Leu Leu Cys Ala Gly
145                 150                 155                 160

Ile Thr Thr Tyr Ser Pro Leu Arg His Trp Gln Ala Gly Pro Gly Lys
                165                 170                 175

Lys Val Gly Val Val Gly Ile Gly Gly Leu Gly His Met Gly Ile Lys
            180                 185                 190

Leu Ala His Ala Met Gly Ala His Val Val Ala Phe Thr Thr Ser Glu
        195                 200                 205

Ala Lys Arg Glu Ala Ala Lys Ala Leu Gly Ala Asp Glu Val Val Asn
    210                 215                 220

Ser Arg Asn Ala Asp Glu Met Ala Ala His Leu Lys Ser Phe Asp Phe
225                 230                 235                 240

Ile Leu Asn Thr Val Ala Ala Pro His Asn Leu Asp Asp Phe Thr Thr
                245                 250                 255

Leu Leu Lys Arg Asp Gly Thr Met Thr Leu Val Gly Ala Pro Ala Thr
            260                 265                 270

Pro His Lys Ser Pro Glu Val Phe Asn Leu Ile Met Lys Arg Arg Ala
        275                 280                 285

Ile Ala Gly Ser Met Ile Gly Gly Ile Pro Glu Thr Gln Glu Met Leu
    290                 295                 300

Asp Phe Cys Ala Glu His Gly Ile Val Ala Asp Ile Glu Met Ile Arg
305                 310                 315                 320

Ala Asp Gln Ile Asn Glu Ala Tyr Glu Arg Met Leu Arg Gly Asp Val
                325                 330                 335

Lys Tyr Arg Phe Val Ile Asp Asn Arg Thr Leu Thr Asp
            340                 345

<210>  5
<211>  789
<212>  DNA
<213>  Ralstonia sp.

<400>  5
atggctagca gaggatcgca tcaccatcac catcacggcg cctatcgact attaaacaaa      60

acagccgtca taaccggtgg aaacagcggc attggcctcg ccacagcgaa gcgcttcgtt     120

gccgagggtg cctatgtatt cattgtcggt cgccggcgga aggaactcga gcaggcggcc     180

gcagaaatcg gtcggaatgt cacggcggtc aaagccgatg tgacaaagct tgaagacctg     240

gaccgacttt acgcgattgt gcgtgagcaa cggggtagca tcgacgtact atttgcgaat     300

tccggcgcaa tcgagcaaaa gacgcttgag gagattactc cggaacacta tgacaggact     360

ttcgatgtca acgttcgggg attgatcttc accgtgcaga aggcacttcc tctgctgcga     420

gacggcggca gcgtgatcct gacaagctcg gtagccggcg tcctaggatt acaggcgcac     480

gacacgtata gtgccgccaa ggcagcggta aggtcgctcg cgaggacatg gaccactgag     540

ttgaaaggtc gcagcattcg tgtcaacgcg gtaagcccag gggcgatcga cacgcctatc      600

atagaaaacc aggtctctac acaggaagaa gctgacgagc tgcgtgcgaa atttgcagct     660

gcgacgcccc tgggtcgcgt cggacgacct gaagagctgg cagcggccgt gttatttctt     720

gcatcggacg acagtagcta cgtagccggc attgagctgt ttgtggacgg tggattgacc     780

caggtctaa                                                             789

<210>  6

<211>  459
<212>  PRT
<213>  Chromobacterium violaceum ATCC 12472

<400>  6

Met Gln Lys Gln Arg Thr Thr Ser Gln Trp Arg Glu Leu Asp Ala Ala
1               5                   10                  15

His His Leu His Pro Phe Thr Asp Thr Ala Ser Leu Asn Gln Ala Gly
            20                  25                  30

Ala Arg Val Met Thr Arg Gly Glu Gly Val Tyr Leu Trp Asp Ser Glu
        35                  40                  45

Gly Asn Lys Ile Ile Asp Gly Met Ala Gly Leu Trp Cys Val Asn Val
    50                  55                  60

Gly Tyr Gly Arg Lys Asp Phe Ala Glu Ala Ala Arg Arg Gln Met Glu
65                  70                  75                  80

Glu Leu Pro Phe Tyr Asn Thr Phe Phe Lys Thr Thr His Pro Ala Val
                85                  90                  95

Val Glu Leu Ser Ser Leu Leu Ala Glu Val Thr Pro Ala Gly Phe Asp
            100                 105                 110

Arg Val Phe Tyr Thr Asn Ser Gly Ser Glu Ser Val Asp Thr Met Ile
        115                 120                 125

Arg Met Val Arg Arg Tyr Trp Asp Val Gln Gly Lys Pro Glu Lys Lys
    130                 135                 140

Thr Leu Ile Gly Arg Trp Asn Gly Tyr His Gly Ser Thr Ile Gly Gly
145                 150                 155                 160

Ala Ser Leu Gly Gly Met Lys Tyr Met His Glu Gln Gly Asp Leu Pro
                165                 170                 175

Ile Pro Gly Met Ala His Ile Glu Gln Pro Trp Trp Tyr Lys His Gly
            180                 185                 190

Lys Asp Met Thr Pro Asp Glu Phe Gly Val Val Ala Ala Arg Trp Leu
        195                 200                 205

Glu Glu Lys Ile Leu Glu Ile Gly Ala Asp Lys Val Ala Ala Phe Val
    210                 215                 220

Gly Glu Pro Ile Gln Gly Ala Gly Gly Val Ile Val Pro Pro Ala Thr
225                 230                 235                 240

Tyr Trp Pro Glu Ile Glu Arg Ile Cys Arg Lys Tyr Asp Val Leu Leu
                245                 250                 255

Val Ala Asp Glu Val Ile Cys Gly Phe Gly Arg Thr Gly Glu Trp Phe
            260                 265                 270

Gly His Gln His Phe Gly Phe Gln Pro Asp Leu Phe Thr Ala Ala Lys
        275                 280                 285

Gly Leu Ser Ser Gly Tyr Leu Pro Ile Gly Ala Val Phe Val Gly Lys
    290                 295                 300

Arg Val Ala Glu Gly Leu Ile Ala Gly Gly Asp Phe Asn His Gly Phe
305                 310                 315                 320

Thr Tyr Ser Gly His Pro Val Cys Ala Ala Val Ala His Ala Asn Val
                325                 330                 335

Ala Ala Leu Arg Asp Glu Gly Ile Val Gln Arg Val Lys Asp Asp Ile
            340                 345                 350

Gly Pro Tyr Met Gln Lys Arg Trp Arg Glu Thr Phe Ser Arg Phe Glu
        355                 360                 365

His Val Asp Asp Val Arg Gly Val Gly Met Val Gln Ala Phe Thr Leu
    370                 375                 380

Val Lys Asn Lys Ala Lys Arg Glu Leu Phe Pro Asp Phe Gly Glu Ile
385                 390                 395                 400

Gly Thr Leu Cys Arg Asp Ile Phe Phe Arg Asn Asn Leu Ile Met Arg
                405                 410                 415

Ala Cys Gly Asp His Ile Val Ser Ala Pro Pro Leu Val Met Thr Arg
            420                 425                 430

Ala Glu Val Asp Glu Met Leu Ala Val Ala Glu Arg Cys Leu Glu Glu
        435                 440                 445

Phe Glu Gln Thr Leu Lys Ala Arg Gly Leu Ala
    450                 455

<210>  7
<211>  452
<212>  PRT
<213>  Pseudomonas putida

<400>  7

Met Ser Glu Gln Asn Ser Gln Thr Gln Ala Trp Gln Ala Leu Ser Arg

1               5                   10                  15

Asp His His Leu Ala Pro Phe Ser Asp Val Lys Gln Leu Ala Glu Lys
            20                  25                  30

Gly Pro Arg Ile Ile Thr Ser Ala Lys Gly Val Tyr Leu Trp Asp Ser
        35                  40                  45

Glu Gly Asn Lys Ile Leu Asp Gly Met Ala Gly Leu Trp Cys Val Ala
    50                  55                  60

Val Gly Tyr Gly Arg Asp Glu Leu Ala Glu Val Ala Ser Gln Gln Met
65                  70                  75                  80

Lys Gln Leu Pro Tyr Tyr Asn Leu Phe Phe Gln Thr Ala His Pro Pro
                85                  90                  95

Ala Leu Glu Leu Ala Lys Ala Ile Ala Asp Val Ala Pro Gln Gly Met
            100                 105                 110

Asn His Val Phe Phe Thr Gly Ser Gly Ser Glu Gly Asn Asp Thr Val
        115                 120                 125

Leu Arg Met Val Arg His Tyr Trp Ala Leu Lys Gly Lys Lys Asn Lys
    130                 135                 140

Asn Val Ile Ile Gly Arg Ile Asn Gly Tyr His Gly Ser Thr Val Ala
145                 150                 155                 160

Gly Ala Ala Leu Gly Gly Met Ser Gly Met His Gln Gln Gly Gly Val
                165                 170                 175

Ile Pro Asp Ile Val His Ile Pro Gln Pro Tyr Trp Phe Gly Glu Gly
            180                 185                 190

Gly Asp Met Thr Glu Ala Asp Phe Gly Val Trp Ala Ala Glu Gln Leu
        195                 200                 205

Glu Lys Lys Ile Leu Glu Val Gly Val Asp Asn Val Ala Ala Phe Ile
    210                 215                 220

Ala Glu Pro Ile Gln Gly Ala Gly Gly Val Ile Ile Pro Pro Gln Thr
225                 230                 235                 240

Tyr Trp Pro Lys Val Lys Glu Ile Leu Ala Arg Tyr Asp Ile Leu Phe
                245                 250                 255

Val Ala Asp Glu Val Ile Cys Gly Phe Gly Arg Thr Gly Glu Trp Phe
            260                 265                 270

Gly Thr Asp Tyr Tyr Asp Leu Lys Pro Asp Leu Met Thr Ile Ala Lys
            275                 280                 285

Gly Leu Thr Ser Gly Tyr Ile Pro Met Gly Gly Val Ile Val Arg Asp
    290                 295                 300

Glu Val Ala Lys Val Ile Ser Glu Gly Gly Asp Phe Asn His Gly Phe
305                 310                 315                 320

Thr Tyr Ser Gly His Pro Val Ala Ala Ala Val Gly Leu Glu Asn Leu
                325                 330                 335

Arg Ile Leu Arg Asp Glu Gln Ile Ile Gln Gln Val His Asp Lys Thr
            340                 345                 350

Ala Pro Tyr Leu Gln Gln Arg Leu Arg Glu Leu Ala Asp His Pro Leu
        355                 360                 365

Val Gly Glu Val Arg Gly Leu Gly Met Leu Gly Ala Ile Glu Leu Val
    370                 375                 380

Lys Asp Lys Ala Thr Arg Ala Arg Tyr Glu Gly Lys Gly Val Gly Met
385                 390                 395                 400

Ile Cys Arg Gln His Cys Phe Asp Asn Gly Leu Ile Met Arg Ala Val
                405                 410                 415

Gly Asp Thr Met Ile Ile Ala Pro Pro Leu Val Ile Ser Val Glu Glu
            420                 425                 430

Ile Asp Glu Leu Val Gln Lys Ala Arg Lys Cys Leu Asp Leu Thr Tyr
        435                 440                 445

Glu Ala Val Arg
    450

<210>  8
<211>  452
<212>  PRT
<213>  Pseudomonas putida

<400>  8

Met Ser Glu Gln Asn Ser Gln Thr Gln Ala Trp Gln Ala Leu Ser Arg
1               5                   10                  15

Asp His His Leu Ala Pro Phe Ser Asp Val Lys Gln Leu Ala Glu Lys
            20                  25                  30

Gly Pro Arg Ile Ile Thr Ser Ala Lys Gly Val Tyr Leu Trp Asp Ser
        35                  40                  45

Glu Gly Asn Lys Ile Leu Asp Gly Met Ala Gly Leu Trp Cys Val Ala
    50                  55                  60

Val Gly Tyr Gly Arg Asp Glu Leu Ala Glu Val Ala Ser Gln Gln Met
65                  70                  75                  80

Lys Gln Leu Pro Tyr Tyr Asn Leu Phe Phe Gln Thr Ala His Pro Pro
                85                  90                  95

Ala Leu Glu Leu Ala Lys Ala Ile Ala Asp Val Ala Pro Gln Gly Met
            100                 105                 110

Asn His Val Phe Phe Thr Gly Ser Gly Ser Glu Gly Asn Asp Thr Val
        115                 120                 125

Leu Arg Met Val Arg His Tyr Trp Ala Leu Lys Gly Lys Lys Asn Lys
    130                 135                 140

Asn Val Ile Ile Gly Arg Ile Asn Gly Tyr His Gly Ser Thr Val Ala
145                 150                 155                 160

Gly Ala Ala Leu Gly Gly Met Ser Gly Met His Gln Gln Gly Gly Val
                165                 170                 175

Ile Pro Asp Ile Val His Ile Pro Gln Pro Tyr Trp Phe Gly Glu Gly
            180                 185                 190

Gly Asp Met Thr Glu Ala Asp Phe Gly Val Trp Ala Ala Glu Gln Leu
        195                 200                 205

Glu Lys Lys Ile Leu Glu Val Gly Val Asp Asn Val Ala Ala Phe Ile
    210                 215                 220

Ala Glu Pro Ile Gln Gly Ala Gly Gly Val Ile Ile Pro Pro Gln Thr
225                 230                 235                 240

Tyr Trp Pro Lys Val Lys Glu Ile Leu Ala Arg Tyr Asp Ile Leu Phe
                245                 250                 255

Val Ala Asp Glu Val Ile Cys Gly Phe Gly Arg Thr Gly Glu Trp Phe
            260                 265                 270

Gly Thr Asp Tyr Tyr Asp Leu Lys Pro Asp Leu Met Thr Ile Ala Lys
        275                 280                 285

Gly Leu Thr Ser Gly Tyr Ile Pro Met Gly Gly Val Ile Val Arg Asp

290                 295                 300

Glu Val Ala Lys Val Ile Ser Glu Gly Gly Asp Phe Asn His Gly Phe
305                 310                 315                 320

Thr Tyr Ser Gly His Pro Val Ala Ala Ala Val Gly Leu Glu Asn Leu
                325                 330                 335

Arg Ile Leu Arg Asp Glu Gln Ile Ile Gln Gln Val His Asp Lys Thr
            340                 345                 350

Ala Pro Tyr Leu Gln Gln Arg Leu Arg Glu Leu Ala Asp His Pro Leu
        355                 360                 365

Val Gly Glu Val Arg Gly Leu Gly Met Leu Gly Ala Ile Glu Leu Val
    370                 375                 380

Lys Asp Lys Ala Thr Arg Ala Arg Tyr Glu Gly Lys Gly Val Gly Met
385                 390                 395                 400

Ile Cys Arg Gln His Cys Phe Asp Asn Gly Leu Ile Met Arg Ala Val
                405                 410                 415

Gly Asp Thr Met Ile Ile Ala Pro Pro Leu Val Ile Ser Val Glu Glu
            420                 425                 430

Ile Asp Glu Leu Val Gln Lys Ala Arg Lys Cys Leu Asp Leu Thr Tyr
        435                 440                 445

Glu Ala Val Arg
    450

<210>  9
<211>  453
<212>  PRT
<213>  Pseudomonas putida

<400>  9

Met Ser Val Asn Asn Pro Gln Thr Arg Glu Trp Gln Thr Leu Ser Gly
1               5                   10                  15

Glu His His Leu Ala Pro Phe Ser Asp Tyr Lys Gln Leu Lys Glu Lys
            20                  25                  30

Gly Pro Arg Ile Ile Thr Lys Ala Gln Gly Val His Leu Trp Asp Ser
        35                  40                  45

Glu Gly His Lys Ile Leu Asp Gly Met Ala Gly Leu Trp Cys Val Ala
    50                  55                  60

Val Gly Tyr Gly Arg Glu Glu Leu Val Gln Ala Ala Glu Lys Gln Met
65                  70                  75                  80

Arg Glu Leu Pro Tyr Tyr Asn Leu Phe Phe Gln Thr Ala His Pro Pro
                85                  90                  95

Ala Leu Glu Leu Ala Lys Ala Ile Thr Asp Val Ala Pro Gln Gly Met
            100                 105                 110

Thr His Val Phe Phe Thr Gly Ser Gly Ser Glu Gly Asn Asp Thr Val
        115                 120                 125

Leu Arg Met Val Arg His Tyr Trp Ala Leu Lys Gly Lys Pro His Lys
    130                 135                 140

Gln Thr Ile Ile Gly Arg Ile Asn Gly Tyr His Gly Ser Thr Phe Ala
145                 150                 155                 160

Gly Ala Cys Leu Gly Gly Met Ser Gly Met His Glu Gln Gly Gly Leu
                165                 170                 175

Pro Ile Pro Gly Ile Val His Ile Pro Gln Pro Tyr Trp Phe Gly Glu
            180                 185                 190

Gly Gly Asp Met Thr Pro Asp Ala Phe Gly Val Trp Ala Ala Glu Gln
        195                 200                 205

Leu Glu Lys Lys Ile Leu Glu Val Gly Glu Asp Asn Val Ala Ala Phe
    210                 215                 220

Ile Ala Glu Pro Ile Gln Gly Ala Gly Gly Val Ile Ile Pro Pro Glu
225                 230                 235                 240

Thr Tyr Trp Pro Lys Val Lys Glu Ile Leu Ala Lys Tyr Asp Ile Leu
                245                 250                 255

Phe Val Ala Asp Glu Val Ile Cys Gly Phe Gly Arg Thr Gly Glu Trp
            260                 265                 270

Phe Gly Ser Asp Tyr Tyr Asp Leu Lys Pro Asp Leu Met Thr Ile Ala
        275                 280                 285

Lys Gly Leu Thr Ser Gly Tyr Ile Pro Met Gly Gly Val Ile Val Arg
    290                 295                 300

Asp Thr Val Ala Lys Val Ile Ser Glu Gly Gly Asp Phe Asn His Gly
305                 310                 315                 320

Phe Thr Tyr Ser Gly His Pro Val Ala Ala Ala Val Gly Leu Glu Asn
                325                 330                 335

Leu Arg Ile Leu Arg Asp Glu Lys Ile Val Glu Lys Ala Arg Thr Glu
            340                 345                 350

Ala Ala Pro Tyr Leu Gln Lys Arg Leu Arg Glu Leu Gln Asp His Pro
        355                 360                 365

Leu Val Gly Glu Val Arg Gly Leu Gly Met Leu Gly Ala Ile Glu Leu
    370                 375                 380

Val Lys Asp Lys Ala Thr Arg Ser Arg Tyr Glu Gly Lys Gly Val Gly
385                 390                 395                 400

Met Ile Cys Arg Thr Phe Cys Phe Asp Asn Gly Leu Ile Met Arg Ala
                405                 410                 415

Val Gly Asp Thr Met Ile Ile Ala Pro Pro Leu Val Ile Ser His Ala
            420                 425                 430

Glu Ile Asp Glu Leu Val Glu Lys Ala Arg Lys Cys Leu Asp Leu Thr
        435                 440                 445

Leu Glu Ala Ile Asn
    450

<210> 10
<211> 455
<212> PRT
<213> Rhodobacter spaeroides

<400> 10

Met Arg Asp Asp Ala Pro Asn Ser Trp Glu Ser Arg Ala Asp Ala Ser
1               5                   10                  15

Ser Phe Tyr Gly Phe Thr Asp Leu Pro Ser Val His Gln Arg Gly Thr
            20                  25                  30

Val Val Leu Thr His Gly Lys Gly Pro Tyr Ile Tyr Asp Val His Gly
        35                  40                  45

Arg Ala Tyr Leu Asp Ala Asn Ser Gly Leu Trp Asn Met Val Ala Gly
    50                  55                  60

Phe Asp His Pro Gly Leu Ile Glu Ala Ala Lys Ala Gln Tyr Glu Arg
65                  70                  75                  80

Phe Pro Gly Tyr His Ala Phe Phe Gly Arg Met Ser Asp Gln Thr Val
                85                  90                  95

Met Leu Ser Glu Lys Leu Val Glu Val Ser Pro Phe Ala Arg Gly Arg
        100                 105                 110

Val Phe Tyr Thr Asn Ser Gly Ser Glu Ala Asn Asp Thr Met Val Lys
        115                 120                 125

Met Leu Trp Phe Leu Gly Ala Ala Glu Gly His Pro Glu Arg Arg Lys
    130                 135                 140

Ile Ile Thr Arg Val Asn Ser Tyr His Gly Val Thr Ala Val Ser Ala
145                 150                 155                 160

Ser Met Thr Gly Lys Pro Tyr Asn Ser Leu Phe Gly Leu Pro Leu Pro
                165                 170                 175

Gly Phe Ile His Val Gly Cys Pro His Tyr Trp Arg Phe Gly Gln Ala
            180                 185                 190

Gly Glu Thr Glu Ala Glu Phe Thr Gln Arg Leu Ala Arg Glu Leu Glu
        195                 200                 205

Ala Thr Ile Ile Lys Glu Gly Pro Asp Thr Ile Ala Gly Phe Phe Ala
    210                 215                 220

Glu Pro Val Met Gly Ala Gly Gly Val Ile Pro Pro Ser Glu Gly Tyr
225                 230                 235                 240

Phe Gln Ala Val Gln Pro Val Leu Lys Arg Tyr Gly Ile Pro Leu Ile
                245                 250                 255

Ala Asp Glu Val Ile Cys Gly Phe Gly Arg Thr Gly Asn Thr Trp Gly
            260                 265                 270

Cys Gln Thr Tyr Asp Phe Met Pro Asp Gly Ile Ile Ser Ser Lys Asn
        275                 280                 285

Ile Thr Ala Gly Phe Phe Pro Met Gly Ala Val Ile Leu Gly Pro Glu
    290                 295                 300

Leu Ala Asp Arg Leu Gln Ala Ala Ser Glu Ala Val Glu Glu Phe Pro
305                 310                 315                 320

His Gly Phe Thr Ala Ser Gly His Pro Val Gly Cys Ala Ile Ala Leu
                325                 330                 335

Lys Ala Ile Asp Val Val Met Asn Glu Gly Leu Ala Glu Asn Val Arg
            340                 345                 350

Ala Leu Thr Pro Lys Phe Glu Ala Gly Leu Ala Tyr Leu Ala Glu Asn
        355                 360                 365

Pro Asn Ile Gly Glu Trp Arg Gly Lys Gly Leu Met Gly Ala Leu Glu
    370                 375                 380

Ala Val Lys Asp Lys Ala Thr Lys Thr Pro Phe Pro Gly Asp Leu Ser
385                 390                 395                 400

Val Ser Glu Arg Ile Ala Asn Ser Cys Thr Asp His Gly Leu Ile Cys
                405                 410                 415

Arg Pro Leu Gly Gln Ser Ile Val Leu Cys Pro Pro Phe Ile Met Thr
            420                 425                 430

Glu Ala Gln Met Asp Glu Met Phe Glu Lys Leu Gly Ala Ala Leu Lys
        435                 440                 445

Lys Val Phe Ala Glu Val Ala
    450                 455

<210>  11
<211>  23
<212>  DNA
<213>  artificial

<220>
<223>  primer

<400>  11
gccatcatag gggttcctaa aga                                              23

<210>  12
<211>  30
<212>  DNA
<213>  artificial

<220>
<223>  primer

<400>  12
actgatggta ccttaagcac ccgccacaga                                       30

<210>  13
<211>  33
<212>  DNA
<213>  artificial

<220>
<223>  primer

<400>  13
gtgacgatca ttgcccgtaa cgcagaccgc ttg                                   33

<210>  14
<211>  33
<212>  DNA
<213>  artificial

<220>
<223>  primer

<400>  14
caagcggtct gcgttacggg caatgatcgt cac                                    33


<210>  15
<211>  27
<212>  DNA
<213>  artificial

<220>
<223>  primer

<400>  15
gcctatcgac tattaaacaa aacagcc                                           27


<210>  16
<211>  30
<212>  DNA
<213>  artificial

<220>
<223>  primer

<400>  16
actgatggta ccttagacct gggtcaatcc                                        30


<210>  17
<211>  1401
<212>  DNA
<213>  Paracoccus denitrificans

<400>  17
atggctagca gaggatcgca tcaccatcac catcacggcg ccaaccaacc gcaaagctgg      60

gaagcccggg ccgagaccta ttcgctctac ggtttcaccg acatgccctc ggtccatcag     120

cggggcacgg tcgtcgtgac ccatggcgag gggccctata tcgtcgatgt ccatggccgc     180

cgctatctgg atgccaattc gggcctgtgg aacatggtcg cgggcttcga ccacaagggc     240

ctgatcgagg ccgccaaggc gcaatacgac cgctttcccg gctatcacgc ctttttcggc     300

cgcatgtccg accagaccgt gatgctgtcg gaaaagctgg tcgaggtctc gccattcgac     360

aacggccggg tcttctatac caattccggc tccgaggcga acgacaccat ggtcaagatg     420

ctgtggttcc tgcatgccgc cgagggcaag ccgcaaaagc gcaagatcct gacgcgctgg     480

aacgcctatc acggcgtgac cgcggtttcg gcctcgatga ccggcaagcc ctacaactcg     540

gtcttcggcc tgccgctgcc cggcttcatc cacctgacct gcccgcatta ctggcgctat     600

ggcgaggaag gcgagaccga ggcgcaattc gtcgcccgcc tggcacgcga gcttgaggat     660

accatcaccc gcgagggcgc cgacaccatc gccggcttct tcgccgagcc ggtgatgggc     720

gcgggggggg tgatcccgcc ggcgaagggt tatttccagg ccatcctgcc gatcttgcgc 780

aagtatgaca tcccgatgat ctcggacgag gtgatctgcg gcttcgggcg caccggcaac 840

acctggggct gcctgaccta cgacttcatg cccgatgcga tcatctcgtc caagaacctg 900

actgcgggct tcttcccgat gggcgccgtc atcctcgggc ccgacctcgc caagcgggtc 960

gaggccgcgg tcgaggcgat cgaggagttc ccgcacggct tcaccgcctc gggccatccg 1020

gtcggctgcg ccatcgcgct gaaggccatc gacgtggtga tgaacgaggg gctggccgag 1080

aatgtccgcc gcctcgcacc ccgcttcgag gcggggctga agcgcatcgc cgaccgcccg 1140

aacatcggcg aataccgcgg catcggcttc atgtgggcgc tggaggcggt caaggacaag 1200

ccgaccaaga cccccttcga cgccaatctt tcggtcagcg agcgcatcgc caatacctgc 1260

accgatctgg ggctgatctg ccggccgctg ggccagtcca tcgtgctgtg cccgcccttc 1320

atcctgaccg aggcgcagat ggacgagatg ttcgaaaagc tggaaaaggc gctcgacaag 1380

gtctttgccg aggtggccta a 1401

<210> 18
<211> 23
<212> DNA
<213> artificial

<220>
<223> primer

<400> 18
gccaaccaac cgcaaagctg gga 23

<210> 19
<211> 33
<212> DNA
<213> artificial

<220>
<223> primer

<400> 19
actgataagc ttttaggcca cctcggcaaa gac 33

<210> 20
<211> 24
<212> DNA
<213> artificial

<220>
<223> primer

<400> 20
ctgccggcca atgggccagt ccat 24

<210> 21
<211> 24
<212> DNA

<213>  artificial

<220>
<223>  primer

<400>  21
atggactggc ccattggccg gcag                                              24


<210>  22
<211>  391
<212>  PRT
<213>  Artificial

<220>
<223>  Bacillus subitilis protein mit His-Tag

<400>  22

Met Ala Ser Arg Gly Ser His His His His His His Gly Ala Ile Ile
1               5                   10                  15

Gly Val Pro Lys Glu Ile Lys Asn Asn Glu Asn Arg Val Ala Leu Thr
            20                  25                  30

Pro Gly Gly Val Ser Gln Leu Ile Ser Asn Gly His Arg Val Leu Val
        35                  40                  45

Glu Thr Gly Ala Gly Leu Gly Ser Gly Phe Glu Asn Glu Ala Tyr Glu
    50                  55                  60

Ser Ala Gly Ala Glu Ile Ile Ala Asp Pro Lys Gln Val Trp Asp Ala
65                  70                  75                  80

Glu Met Val Met Lys Val Lys Glu Pro Leu Pro Glu Glu Tyr Val Tyr
                85                  90                  95

Phe Arg Lys Gly Leu Val Leu Phe Thr Tyr Leu His Leu Ala Ala Glu
            100                 105                 110

Pro Glu Leu Ala Gln Ala Leu Lys Asp Lys Gly Val Thr Ala Ile Ala
        115                 120                 125

Tyr Glu Thr Val Ser Glu Gly Arg Thr Leu Pro Leu Leu Thr Pro Met
    130                 135                 140

Ser Glu Val Ala Gly Arg Met Ala Ala Gln Ile Gly Ala Gln Phe Leu
145                 150                 155                 160

Glu Lys Pro Lys Gly Gly Lys Gly Ile Leu Leu Ala Gly Val Pro Gly
                165                 170                 175

Val Ser Arg Gly Lys Val Thr Ile Ile Gly Gly Gly Val Val Gly Thr
            180                 185                 190

Asn Ala Ala Lys Met Ala Val Gly Leu Gly Ala Asp Val Thr Ile Ile
        195                 200                 205

Ala Arg Asn Ala Asp Arg Leu Arg Gln Leu Asp Asp Ile Phe Gly His
    210                 215                 220

Gln Ile Lys Thr Leu Ile Ser Asn Pro Val Asn Ile Ala Asp Ala Val
225                 230                 235                 240

Ala Glu Ala Asp Leu Leu Ile Cys Ala Val Leu Ile Pro Gly Ala Lys
                245                 250                 255

Ala Pro Thr Leu Val Thr Glu Glu Met Val Lys Gln Met Lys Pro Gly
            260                 265                 270

Ser Val Ile Val Asp Val Ala Ile Asp Gln Gly Gly Ile Val Glu Thr
        275                 280                 285

Val Asp His Ile Thr Thr His Asp Gln Pro Thr Tyr Glu Lys His Gly
    290                 295                 300

Val Val His Tyr Ala Val Ala Asn Met Pro Gly Ala Val Pro Arg Thr
305                 310                 315                 320

Ser Thr Ile Ala Leu Thr Asn Val Thr Val Pro Tyr Ala Leu Gln Ile
                325                 330                 335

Ala Asn Lys Gly Ala Val Lys Ala Leu Ala Asp Asn Thr Ala Leu Arg
            340                 345                 350

Ala Gly Leu Asn Thr Ala Asn Gly His Val Thr Tyr Glu Ala Val Ala
        355                 360                 365

Arg Asp Leu Gly Tyr Glu Tyr Val Pro Ala Glu Lys Ala Leu Gln Asp
    370                 375                 380

Glu Ser Ser Val Ala Gly Ala
385                 390

<210>  23
<211>  1176
<212>  DNA
<213>  artificial

<220>
<223>  Bacillus subtilis protein mit His-Tag

<400>  23
atggctagca gaggatcgca tcaccatcac catcacggcg ccatcatagg ggttcctaaa        60

gagataaaaa acaatgaaaa ccgtgtcgca ttaacacccg ggggcgtttc tcagctcatt 120

tcaaacggcc accgggtgct ggttgaaaca ggcgcgggcc ttggaagcgg atttgaaaat 180

gaagcctatg agtcagcagg agcggaaatc attgctgatc cgaagcaggt ctgggacgcc 240

gaaatggtca tgaaagtaaa agaaccgctg ccggaagaat atgtttattt tcgcaaagga 300

cttgtgctgt ttacgtacct tcatttagca gctgagcctg agcttgcaca ggccttgaag 360

gataaaggag taactgccat cgcatatgaa acggtcagtg aaggccggac attgcctctt 420

ctgacgccaa tgtcagaggt tgcgggcaga atggcagcgc aaatcggcgc tcaattctta 480

gaaaagccta aaggcggaaa aggcattctg cttgccgggg tgcctggcgt ttcccgcgga 540

aaagtaacaa ttatcggagg aggcgttgtc gggacaaacg cggcgaaaat ggctgtcggc 600

ctcggtgcag atgtgacgat cattgcccgt aacgcagacc gcttgcgcca gcttgatgac 660

atcttcggcc atcagattaa aacgttaatt tctaatccgg tcaatattgc tgatgctgtg 720

gcggaagcgg atctcctcat ttgcgcggta ttaattccgg gtgctaaagc tccgactctt 780

gtcactgagg aaatggtaaa acaaatgaaa cccggttcag ttattgttga tgtagcgatc 840

gaccaaggcg gcatcgtcga aactgtcgac catatcacaa cacatgatca gccaacatat 900

gaaaaacacg gggttgtgca ttatgctgta gcgaacatgc caggcgcagt ccctcgtaca 960

tcaacaatcg ccctgactaa cgttactgtt ccatacgcgc tgcaaatcgc gaacaaaggg 1020

gcagtaaaag cgctcgcaga caatacggca ctgagagcgg gtttaaacac cgcaaacgga 1080

cacgtgacct atgaagctgt agcaagagat ctaggctatg agtatgttcc tgccgagaaa 1140

gctttacagg atgaatcatc tgtggcgggt gcttaa 1176

## Beschreibung

Die vorliegende Erfindung betrifft ein Polypeptid umfassend die Aminosäuresequenz der Alanindehydrogenase (AlaDH) aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist, ein Nukleinsäuremolekül kodierend für eine derartiges Polypeptid sowie ein Verfahren zur Herstellung von Alanin oder einer unter Verbrauch von Alanin entstehenden Verbindung umfassend den Schritt Umsetzen von Pyruvat mit Ammonium und NADPH zu Alanin durch Kontaktieren mit dem erfindungsgemäßen Polypeptid oder der erfindungsgemäßen Zelle.

Amine finden als Synthesebausteine für eine Vielzahl von Produkten der chemischen Industrie wie Epoxidharze, Polyurethanschäume, Isocyanate und insbesondere Polyamide Verwendung. Letztere stellen eine Klasse von Polymeren dar, die durch sich wiederholende Amidgruppen gekennzeichnet sind. Der Begriff "Polyamide" bezeichnet im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die ausgehend von beim Cracken von fossilen Kohlenwasserstoffen anfallenden Alkanen gewonnen werden können. Es können jedoch auch Derivate von Aminen, genauer Aminocarbonsäure, Lactame und Diamine, zur Herstellung von Polymeren auf Amidbasis verwendet werden. Von Interesse sind weiterhin kurzkettige, gasförmige Alkane als Edukte, die ausgehend von nachwachsenden Rohstoffen mittels biotechnologischer Verfahren gewonnen und anschließend aminiert werden können.

Viele kommerziell stark nachgefragte Polyamide werden ausgehend von Lactamen hergestellt. Zum Beispiel kann man "Polyamid 6" durch Polymerisation von ε-Caprolactam und "Polyamid 12" durch Polymerisierung von Laurinlactam erhalten. Weitere kommerziell interessante Produkte umfassen Copolymere von Lactam, z. B. Copolymere von ε-Caprolactam und Laurinlactam.

Die konventionelle chemisch-technische Erzeugung von Aminen ist von der Versorgung mit fossilen Rohstoffen abhängig, ineffizient, und es fallen dabei große Mengen unerwünschter Nebenprodukte an, in manchen Schritten der Synthese bis zu 80 %. Ein Beispiel für einen solchen Prozess stellt die Herstellung von Laurinlactam dar, das konventionell durch Trimerisierung von Butadien gewonnen wird. Das Trimerisierungsprodukt Cyclododekatrien wird hydriert und das daraus resultierende Cyclododekan wird zu Cyclodekanon oxidiert, das anschließend mit Hydroxylamin zu Cyclododekanoxin umgesetzt wird, welche schließlich über eine Beckmann-Umlagerung zu Laurinlactam umgewandelt wird.

Eingedenk dieser Nachteile wurden Verfahren entwickelt, um Amine unter Verwendung von Biokatalysatoren ausgehend von nachwachsenden Rohstoffen zu gewinnen. Dabei werden landwirtschaftlich erzeugte Produkte wie Zucker oder Fettsäuren, die bereits Alkoholfunktionen enthalten können, zum Aldehyd oder Keton oxidiert. In einem nachgeschalteten Schritt wird der Aldehyd oder das Keton anschließend durch eine Transaminase unter Verbrauch einer Aminosäure zum Amin umgesetzt. Die Aminosäure wird vorteilhafterweise durch eine während der Transaminasereaktion anwesende Aminosäuredehydrogenase unter Verbrauch von eines anorganischen Stickstoffsalzes regeneriert.

Beispielsweise beschreibt die Internationale Patentanmeldung PCT/EP 2008/067447 ein biologisches System zur Herstellung von ω-Aminocarbonsäuren unter Verwendung einer Zelle, die eine Reihe von geeigneten enzymatischen Aktivitäten aufweist und in der Lage ist, Carbonsäuren zu entsprechender ω-Aminocarbonsäure umzuwandeln. Insbesondere weist die Zelle das AlkBGT-Oxidasesystem aus *Pseudomonas putida* GPO1 auf, die die ω-Aminocarbonsäure zunächst zur ω-Hydroxycarbonsäure und dann zum Aldehyd oxidiert. Anschließend folgt eine Aminierung des Oxidationsproduktes durch eine ebenfalls exprimierte Transaminase.

Die EP11174729.1 und die EP11006458.1 beschreiben Verfahren zur Herstellung von Aminen ausgehend von Alkanen oder von Alkoholen, die sich dadurch auszeichnen, dass der Alkohol oder das in einem ersten Verfahrensschritt zum Alkohol hydroxylierte Alkan unter durch eine Alkoholdehydrogenase und unter Verbrauch von NAD⁺ zum Aldehyd oder Keton und anschließend unter durch eine Transaminase unter Verbrauch von Alanin als Amindonor zum Amin umgesetzt wird. Das verbrauchte Alanin kann durch eine anwesende Alanindehydrogenase unter Verbrauch von Pyruvat und Ammonium regeneriert werden, so dass insgesamt keine Zufuhr von Alanin erforderlich ist.

Unter den in der Literatur beschriebenen Alanindehydrogenasen sind die Alanindehydrogenase von *Bacillus subtilis* und Varianten davon für ein solches Verfahren geeignet. Nachteilig ist speziell an dieser Gruppe von Alanindehydrogenasen, dass sie NADH-abhängig sind, also einen anderen reduzierten Redoxfaktor verbrauchen als den, den die zahlreiche biotechnologisch besonders bewährte NAD-abhängige Alkoholdehydrogenasen bei der Oxidation des Alkohols generieren.

Dies bedeutet im Falle einer in vitro-Reaktion, dass der Reaktion pro umgesetztes Alkoholmolekül ein NAD⁺- und ein NADPH-Molekül zugeführt werden muss. Wird die Reaktion hingegen *in vivo* unter Verwendung eines Ganzzellkatalysators durchgeführt, so kann die Zelle die jeweiligen Redoxfaktoren zwar grundsätzlich über den Primärstoffwechsel bereitstellen; dies stellt jedoch eine erhebliche Belastung des Metabolismus dar, die eine Verringerung der Ausbeute an Produkt und/oder eine kürzere Verwendbarkeit der Zelle mit sich bringt.

Vor diesem Hintergrund besteht die der Erfindung zu Grunde liegende Aufgabe darin, ein redoxneutrales Enzymsystem zur Aminierung von Alkoholen bereitzustellen, d. h. ein System umfassend Enzyme, die zur Katalyse der Umwandlung vom Alkohol zum Amin unter Regenerierung des Alanins keine Zufuhr von extern hergestelltem NAD⁺ oder NADPH oder sonstigen Redoxfaktoren benötigen.

Eine weitere der Erfindung zu Grunde liegende Aufgabe besteht darin, eine Alanindehydrogenase bereitzustellen, die für die enzymatisch katalysierte Umwandlung vom Alkohol zum Amin in Anwesenheit von NADPH geeignet ist und die Reaktion mit möglichst hoher Wechselzahl, d. h. der Zahl der pro Sekunde umgesetzten Substratmoleküle, katalysiert.

Eine weitere der Erfindung zu Grunde liegende Aufgabe besteht darin, die Alanindehydrogenase aus *Bacillus subtilis* derart zu verändern, dass das Enzym die Umwandlung von Pyruvat zu Alanin mit höherer Affinität zu NADPH als Substrat katalysiert und die Affinität des Enzyms zu NADPH bevorzugt höher ist als die zu NADH, noch bevorzugter ohne dass es zu einer signifikanten Verringerung der Wechselzahl relativ zur Wechselzahl des Wildtypenzyms kommt.

Eine weitere der Erfindung zu Grunde liegende Aufgabe besteht darin, ein redoxneutrales, für eine Eintopfreaktion unter milden Reaktionsbedingungen, d. h. insbesondere solchen ohne extreme Temperaturen oder pH-Werte und in Abwesenheit von Schwermetalle enthaltenden Katalysatoren oder sonstigen toxischen Verbindungen, geeignetes System zur Aminierung von Alkoholen bereitzustellen.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird in einem ersten Aspekt gelöst durch ein Polypeptid umfassend die Aminosäuresequenz der Alanindehydrogenase aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist.

Das Problem wird in einer ersten Ausführungsform des ersten Aspekts durch ein Polypeptid gelöst, wobei es sich bei der Aminosäure mit positiv geladener Seitenkette um Arginin handelt.

Das Problem wird in einer zweiten Ausführungsform des ersten Aspekts, die auch eine Ausführungsform des ersten Aspekts darstellt, gelöst durch ein Polypeptid, wobei zusätzlich Asp196 oder eine an homologer Position in der Aminosäuresequenz befindliche Aminosäure gegen eine Aminosäure mit neutraler oder positiv geladener Seitenkette ausgetauscht ist.

Das Problem wird in einer dritten Ausführungsform des ersten Aspekts, die auch eine Ausführungsform des ersten bis zweiten Aspekts darstellt, durch ein Polypeptid gelöst, wobei es sich bei der Aminosäure mit neutraler oder positiv geladener Seitenkette um eine Aminosäure aus der Gruppe handelt, die Alanin, Glycin, Serin und Cystein umfasst, bevorzugt um Alanin.

Das Problem wird in einer vierten Ausführungsform des ersten Aspekts, die auch eine Ausführungsform des ersten bis zweiten Aspekts darstellt, durch ein Polypeptid gelöst, wobei es sich um das in SEQ ID NO 1 dargestellte Polypeptid handelt.

Das der Erfindung zu Grunde liegende Problem wird in einem zweiten Aspekt gelöst durch ein Nukleinsäuremolekül umfassend eine für das Polypeptid nach dem ersten Aspekt und seiner Ausführungsformen kodierende Nukleotidsequenz.

Das Problem wird in einer ersten Ausführungsform des zweiten Aspekts gelöst durch ein Nukleinsäuremolekül, wobei es sich um die in SEQ ID NO 2 dargestellte Nukleotidsequenz handelt.

Das der Erfindung zu Grunde liegende Problem wird in einem dritten Aspekt gelöst durch einen Vektor umfassend das Nukleinsäuremolekül nach dem zweiten Aspekt und seiner Ausführungsformen.

Das der Erfindung zu Grunde liegende Problem wird in einem vierten Aspekt gelöst durch eine Zelle umfassend das Polypeptid nach dem ersten Aspekt, das Nukleinsäuremolekül nach dem zweiten Aspekt oder den Vektor nach dem dritten Aspekt und der jeweiligen Ausführungsformen.

Das Problem wird in einer ersten Ausführungsform des vierten Aspekts gelöst durch eine Zelle, weiter exprimierend eine NADP⁺-abhängige Alkoholdehydrogenase.

Das Problem wird in einer zweiten Ausführungsform des vierten Aspekts, die auch eine Ausführungsform des ersten Aspekts darstellt, gelöst durch eine Zelle nach dem vierten Aspekt oder der ersten Ausführungsform des vierten Aspekts, weiter exprimierend eine Transaminase.

Das der Erfindung zu Grunde liegende Problem wird in einem vierten Aspekt gelöst durch ein Verfahren zur Herstellung von Alanin oder einer unter Verbrauch von Alanin entstehenden Verbindung, umfassend den Schritt
c) Umsetzen von Pyruvat mit Ammonium und NADPH zu Alanin durch Kontaktieren mit dem Polypeptid nach dem ersten Aspekt oder mit der Zelle nach dem zweiten Aspekt oder einer der jeweiligen Ausführungsformen.

Das Problem wird in einer ersten Ausführungsform des vierten Aspekts gelöst durch Verfahren, weiter umfassend
a) Umsetzen eines primären bzw. sekundären Alkohols zum Aldehyd bzw. Keton durch Kontaktieren mit einer NADP⁺-abhängigen Alkoholdehydrogenase in Anwesenheit von NADP⁺.

Das Problem wird in einer zweiten Ausführungsform des vierten Aspekts, die auch eine Ausführungsform des ersten Aspekts darstellt, gelöst durch ein Verfahren, weiter umfassend
b) Umsetzen des in Schritt a) hergestellten Aldehydes bzw. Ketons zum Amin durch Kontaktieren mit einer Transaminase in Anwesenheit von Alanin.

Das Problem wird in einer dritten Ausführungsform des vierten Aspekts, die auch eine Ausführungsform des ersten bis zweiten Aspekts darstellt, gelöst durch ein Verfahren, wobei die Schritte a), b) und c) in der gleichen Reaktionsmischung ablaufen, bevorzugt gleichzeitig.

Das Problem wird in einer weiteren Ausführungsform des dritten oder vierten Aspekts oder einer Ausführungsform des dritten oder vierten Aspekts gelöst durch eine Zelle oder ein Verfahren, wobei die Zelle sämtliche Enzyme aus der Gruppe umfassend das Polypeptid nach einem der Ansprüche 1 bis 5, die NADP⁺-abhängige Alkoholdehydrogenase und die Transaminase intrazellulär lokalisiert exprimiert.

Das Problem wird in einer weiteren Ausführungsform des dritten oder vierten Aspekts oder einer Ausführungsform des dritten oder vierten Aspekts gelöst durch eine Zelle oder ein Verfahren, wobei es sich bei der NADP⁺-abhängigen Alkoholdehydrogenase um eine NADP⁺-abhängige Alkoholdehydrogenase aus der Gruppe umfassend die Alkoholdehydrogenasen aus *E. coli* (YjgB, Datenbankcode ZP_07117674, und ein YahK, Datenbankcode BAE76108.1) und die Alkoholdehydrogenase aus *Ralstonia* sp. (SEQ ID NO: 3), bevorzugt um die Alkoholdehydrogenase aus *Ralstonia* sp. (SEQ ID NO: 5) oder Varianten davon handelt.

Das Problem wird in einer weiteren Ausführungsform des dritten oder vierten Aspekts oder einer Ausführungsform des dritten oder vierten Aspekts gelöst durch eine Zelle oder ein Verfahren, wobei es sich bei der Transaminase um eine Transaminase aus der Gruppe umfassend die Transaminasen aus *Chromobacterium violaceum* (Datenbankcode NP_901695), *Pseudomonas putida* (Datenbankencode YP_001668026.1 or YP_001671460) und *Rhodobacter sphaeroides* (strain ATCC 17023; Datenbankcode YP_353455) sowie Varianten davon handelt, bevorzugt um die Transaminase aus *Chromobacterium violaceum* (Datenbankcode NP_901695).

Die vorliegende Erfindung basiert auf der überraschenden Erkenntnis der Erfinder, dass ein Enzymsystem umfassend eine Alkoholdehydrogenase, bevorzugt eine NADP⁺-abhängige Alkoholdehydrogenase, eine Transaminase und ein Polypeptid umfassend die Aminosäuresequenz der Alanindehydrogenase aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist, verwendet werden kann, um die Aminierung eines Alkohols redoxneutral zu katalysieren.

Weiterhin basiert die vorliegende Erfindung auf der überraschenden Erkenntnis der Erfinder, dass die Affinität eines Polypeptid umfassend die Aminosäuresequenz der Alanindehydrogenase aus *Bacillus subtilis* zu NADPH dadurch erhöht werden kann, dass Leu197 oder eine an homologer Position in der Aminosäuresequenz befindliche Aminosäure gegen eine Aminosäure mit positiv geladener Seitenkette ausgetauscht ist und das Enzym dennoch katalytisch aktiv bleibt und die Reaktion mit hoher Wechselzahl zu katalysieren vermag.

Die Erfindung betrifft ein Polypeptid umfassend die Aminosäuresequenz der Alanindehydrogenase aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist. Dieses Polypeptid ist im Sequenzprotokoll unter der SEQ ID NO 1 verzeichnet. In einer bevorzugten Ausführungsform wird unter dem Begriff "Alanindehydrogenase", wie hierein verwendet, ein enzymatisch aktives Polypeptid verstanden, das die Umwandlung von Pyruvat unter Verbrauch von Ammonium und NADH bzw. NADPH, bevorzugt NADPH, zu Alanin und NAD⁺ bzw. NADP⁺, bevorzugt NADP⁺, katalysiert. Die Aminosäuresequenz der Alaninde-hydrogenase von *Bacillus subtilis* ist wie die Sequenzen der anderen in dieser Anmeldung durch Angabe eines Datenbankcodes bezeichneten Moleküle oder Sequenzen in der Datenbank des NCBI in der am 1. April 2012 online zugänglichen Version unter dem Datenbankcode L20916 zu finden.

Die erfindungsgemäße Lehre kann jedoch nicht nur unter Verwendung der exakten Aminosäure- oder Nukleinsäuresequenzen bzw. auf die exakten Aminosäure- oder Nukleinsäuresequenzen der hierin beschriebenen biologischen Makromoleküle ausgeführt bzw. angewendet werden, beispielsweise der unter Datenbankcode L20916 beschriebenen Alanindehydrogenase, sondern auch unter Verwendung von bzw. auf Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat durch ein Aspartat oder ein Leucin durch ein Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Alanindehydrogenase enzymatisch aktiven Polypeptids die gleiche oder im Wesentlichen die gleiche proteolytische Aktivität auf, d. h. die Fähigkeit, die Umsetzung von Pyruvat, Ammonium und NAD(P)H, bevorzugt NADPH, zu Alanin zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* 1995 beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5 x SSC-Puffer bei einer Temperatur von ca. 50°C - 68 °C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50 °C - 68 °C, ca. 52 °C - 68 °C, ca. 54 °C - 68 °C, ca. 56 °C - 68 °C, ca. 58 °C - 68 °C, ca. 60 °C - 68 °C, ca. 62 °C - 68 °C, ca. 64 °C - 68 °C, ca. 66 °C - 68 °C eingestellt wird. Temperaturbereiche von ca. 64 °C - 68 °C oder ca. 66 °C - 68 °C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2 °C von 50 °C auf 68 °C können Polynukleotidfragmente isoliert werden, die beispielsweise in der Reihenfolge zunehmender Bevorzugung mindestens 70 % oder mindestens 80 % oder mindestens 90 %, mindestens 91 %, mindestens 92 %, mindestens 93 %, mindestens 94 %, mindestens 95 %, mindestens 96 %, mindestens 97 %, mindestens 98 % oder mindestens 99 % Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Für die Durchführung der erfindungsgemäßen Lehre ist essentiell, dass die verwendete Alanindehydrogenase von *Bacillus subtilis* nicht die Wildtypsequenz des Enzyms umfasst, sondern eine Sequenz, bei der Leu197 oder eine an homologer Position in der Aminosäuresequenz befindliche Aminosäure gegen eine Aminosäure mit positiv geladener Seitenkette ausgetauscht ist. In einer bevorzugten Ausführungsform bedeutet die Bezeichnung "homologe Position", wie hierin verwendet, dass die entsprechende Position bei einem Alignment des untersuchten Moleküls als zur Position X der Aminosäuresequenz aus der Alanindehydrogenase von *Bacillus subtilis* homolog erscheint. Dem Fachmann sind zahlreiche Softwarepakete und Algorithmen bekannt, mit denen ein Alignment von Aminosäuresequenzen angefertigt werden kann. Beispielhafte Softwarepakete Verfahren umfassen das vom EMBL bereitgestellte Paket ClustalW (Larkin *et al.,* 2007; Goujon *et al.* 2010) oder sind in Arthur M.

Lesk (2008), Introduction to Bioinformatics, 3rd edition, aufgeführt und beschrieben. In einer bevorzugten Ausführungsform wird unter dem Begriff "Aminosäure" bzw. "Alanin", eine proteinogene L-Aminosäure bzw. L-Alanin verstanden, d.h. eine in der Natur von Organismen universell zum Aufbau von Polypeptiden verwendete Aminosäure. Der Begriff "Aminosäure" umfasst, wie sämtliche in dieser Anmeldung genannten Verbindungen, auch sämtliche Kristall-, Salz- oder ähnliche Formen der entsprechenden Verbindung. Beispielsweise umfasst Histidin auch das Salz aus protoniertem Histidin und einem Chloridion.

Für die Aktivität der Alanindehydrogenase ist essentiell, dass die Edukte der katalysierten Reaktion in ausreichender Menge vorliegen, insbesondere eine anorganische Stickstoffquelle. In einer bevorzugten Ausführungsform ist unter dem Begriff "anorganische Stickstoffquelle", wie hierin verwendet, ein anorganisches stickstoffhaltiges Salz zu verstehen, das Ammonium umfasst oder im Stoffwechsel der Zelle zu Ammonium umgewandelt werden kann. Beispiele umfassen Ammoniumchlorid, Ammoniumnitrat, Ammoniumsulfat, Ammoniumhydroxid, Ammoniumphosphat, Ammoniumcarbonat und dergleichen. In einer bevorzugten Ausführungsform beträgt die Konzentration von Ammonium im Medium 0,05 bis 5, bevorzugter 0,1 bis 3, am bevorzugtesten 0,5 bis 3 g/L. Bevorzugt wird die anorganische Stickstoffquelle der Zelle erfindungsgemäß dadurch bereitgestellt, dass die entsprechende Verbindung in ausreichender Menge zur wässrigen Phase gegeben wird, in der die Reaktion abläuft.

Aminosäuren mit einer positiv geladenen Seitenketten umfassen die Aminosäuren Arginin, Lysin und Histidin, bevorzugt Arginin und Lysin, am bevorzugtesten Arginin. Mögliche erfindungsgemäße Polypeptide weisen beispielsweise die Mutationen Leu197Arg, Leu197Lys und Leu197His auf. In einer bevorzugten Ausführungsform umfasst die erfindungsgemäße Lehre ausdrücklich auch Alanindehydrogenasen aus anderen Organismen als *Bacillus subtilis,* sofern diese zur Gruppe der Varianten der Alanindehydrogenase von *Bacillus subtilis* zählen.

In einer weiteren bevorzugten Ausführungsform umfasst die erfindungsgemäße Lehre auch ein Polypeptid, das neben dem erwähnten Austausch an der Position des Leu197 einen weiteren Austausch an der Position Asp196 oder an dazu homologer Position aufweist, nämlich gegen eine Aminosäure mit neutraler oder positiver geladener Seitenkette. In einer bevorzugten Ausführungsform umfassen Aminosäuren mit neutraler Seitenkette die Gruppe umfassend Glycin, Alanin, Valin, Leucin, Tryptophan, Tyrosin, Isoleucin, Serin, Cystein, Threonin, Glutamin, Methionin, Phenylalanin, Prolin und Asparagin. In einer bevorzugten Ausführungsform umfassen Aminosäuren mit positiv geladener Seitenkette die Gruppe umfassend Arginin, Lysin und Histidin, bevorzugt Arginin und Lysin.

Erfindungsgemäße Nukleinsäuremoleküle umfassen sämtliche unmodifizierte oder modifizierte DNA- und RNA-Moleküle umfassend eine für ein erfindungsgemäßes Polypeptid kodierende Nukleinsäuresequenz. Der Fachmann ist mit Standardverfahren der Molekularbiologie oder der synthetischen Chemie vertraut, mittels derer derartige Nukleinsäuremoleküle hergestellt werden können, beispielsweise der Polymerase-Kettenreaktion oder Festphasensynsthese. In einer bevorzugten Ausführungsform handelt es sich bei dem Nukleinsäuremolekül um ein Nukleinsäuremolekül, das die in SEQ ID NO 2 dargestellte Nukleotidsequenz umfasst oder sie darstellt.

Die erfindungsgemäße Lehre kann sowohl unter Verwendung eines erfindungsgemäßen isolierten Polypeptids, Nukleinsäuremoleküls oder Vektors als auch unter Verwendung einer erfindungsgemäßen Zelle als Ganzzellkatalysator ausgeführt werden. In einer bevorzugten Ausführungsform versteht man unter dem Begriff "Ganzzellkatalysator", wie hierin verwendet, eine intakte, lebensfähige und metabolisch aktive Zelle, die eine erwünschte enzymatische Aktivität bereitstellt. Der Ganzzellkatalysator kann das zu verstoffwechselnde Substrat, im Falle der vorliegenden Erfindung den Alkohol oder das daraus entstehende Oxidationsprodukt, entweder ins Zellinnere transportieren, wo es von cytosolischen Enzymen verstoffwechselt wird, oder er kann das Enzym von Interesse auf seiner Oberfläche präsentieren, wo es gegenüber Substraten im Medium direkt exponiert ist. Dem Fachmann sind zahlreiche Systeme zur Herstellung von Ganzzellkatalysatoren bekannt, beispielsweise aus der DE 60216245. In einer besonders bevorzugten Ausführungsform exprimiert die Zelle das erfindungsgemäße Polypeptid mit NADPH-abhängiger Alanindehydrogenaseaktivität, die Transaminase und/oder die NADP⁺-abhängige Alkoholdehydrogenase intrazellulär, d. h. das jeweilige Enzym ist nach der Expression dauerhaft im Inneren der Zelle lokalisiert, insbesondere im Cytosol der Zelle oder als Membran- oder in der Membran verankertes Protein, das ins Cytosol der Zelle hineinragt.

In einer bevorzugten Ausführungsform handelt es sich bei der als Ganzzellkatalysator oder bei der als Expressionssystem verwendeten Zelle um eine prokaryotische, bevorzugt um eine bakterielle Zelle. In einer weiteren bevorzugten Ausführungsform handelt es sich um eine Säugetierzelle. In einer weiteren bevorzugten Ausführungsform handelt es sich um eine niedere eukaryotische Zelle, bevorzugt um eine Hefezelle. Beispielhafte prokaryotische Zellen umfassen *Escherichia,* besonders *Escherichia coli,* und Stämme der Gattung *Pseudomonas* und *Corynebacterium.* Beispielhafte niedere eukaryotische Zellen umfassen die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.*

Im Falle der Verwendung eines erfindungsgemäßen Ganzzellkatalysators kann bei einigen Substraten, insbesondere solchen mit langen Alkylketten, der Eintritt des Moleküls ins Innere des Ganzzellkatalysators für die Herstellung der erwünschten Substanz limitierend sein. Im Falle langkettiger Alkane und Alkohole ist bevorzugt, dass der Ganzzellkatalysator ein AlkL-Polypeptid aufweist. In einer bevorzugten Ausführungsform handelt es sich bei einem "AlkL-Polypeptid", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um ein in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Bei den erfindungsgemäß eingesetzten enzymatisch aktiven Polypeptiden, insbesondere Alkoholdehydrogenase, Transaminase und Alanindehydrogenase, kann es sich aber jeweils auch um eine Präparation des erfindungsgemäßen Polypeptides in sämtlichen Aufreinigungsstufen, vom kruden Lysat bis zum isolierten Polypeptid, handeln. Die Zelle kann eine oder mehr als eine für ein erfindungsgemäß verwendetes Enzym kodierende Nukleinsäuresequenz auf einem Plasmid oder integriert in ihr Genom enthalten. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden, beispielsweise Vektoren vom Typ pET oder pGEX. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltose-bindendes Protein.

Für eine Reihe von Anwendungen empfiehlt sich ausdrücklich die Verwendung des erfindungsgemäßen Polypeptides in isolierter Form. In einer bevorzugten Ausführungsform bedeutet der Begriff "isoliert", wie hierin verwendet, dass das Enzym in reinerer und/oder aufkonzentrierter Form vorliegt als in seiner natürlichen Quelle. In einer bevorzugten Ausführungsform gilt das Enzym als isoliert, wenn es ein Polypeptidenzym ist und mehr als 60, 70, 80, 90 oder bevorzugt 95 % des Massenproteinanteils der entsprechenden Präparation ausmacht. Dem Fachmann sind zahlreiche Verfahren zur Messung der Masse eines Proteins in einer Lösung bekannt, beispielsweise die visuelle Abschätzung anhand der Dicke von entsprechenden Proteinbanden auf SDS-Polyacrylamidgelen, NMR-Spektroskopie oder massenspektrometriebasierte Verfahren.

Die aufgereinigten enzymatisch aktiven Polypeptide können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kopplungs-Chemie (z.B. Kits der Firma Pierce).

Bei den erfindungsgemäß verwendeten Enzymen handelt es sich bevorzugt um rekombinante Enzyme. In einer bevorzugten Ausführungsform wird unter dem Begriff "rekombinant", wie hierin verwendet, verstanden, dass das entsprechende Nukleinsäure-Molekül in der Natur nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen.

Voraussetzung für die Benutzung der erfindungsgemäßen Lehre ist das Vorliegen einer wässrigen Phase, d. h. einem wässrigen Kultur- oder Reaktionsmedium, das zur wenigstens zeitweiligen Erhaltung oder Kultivierung der Zelle oder der Aktivität des erfindungsgemäßen Polypeptides geeignet ist. Dem Fachmann sind zahlreiche wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medium, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässrige Phase", wie hierin verwendet, ein mit hydrophoben Lösungsmitteln im Wesentlichen unmischbares Reaktions- oder Kulturmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, wenigstens zeitweilig erhält oder fördert. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 5 und 42 °C, bevorzugter zwischen 15 und 40 °C, am bevorzugtesten zwischen 20 und 37 °C.

Die Verwendung von Enzymen erfordert die Anwesenheit aller notwendigen Substrate. Beispielsweise erfordert die Aktivität der Alanindehydrogenase die Anwesenheit der Substrate Ammonium, eines geeigneten reduzierten Redoxfaktors und Pyruvat. Weiterhin ist für die Aktivität von Enzymen eine geeignete wässrige Lösung erforderlich, d. h. eine Lösung, die mit Hinblick auf den anwesenden erforderlichen Puffer, pH, Temperatur, Salzkonzentration, die Anwesenheit notwendiger Cofaktoren oder aktivitätsfördernder oder -erhaltender weiterer Polypeptide und sonstige relevante Faktoren geeignet ist, um die Aktivität des Enzyms zumindest zeitweilig zu erhalten. Verfahren zur Auswahl geeigneter Lösungen und enzymatische Tests, mittels derer die Aktivität von Enzymen bestimmt werden kann, sind dem Fachmann bekannt und im Stand der Technik, beispielsweise in Cornish-Bowden (1995), Fundamentals of Enzym Kinetics, Portland Press Limited, beschrieben.

Erfindungsgemäß exprimiert die erfindungsgemäße Zelle neben der modifizierten Alanindehydrogenase aus *Bacillus subtilis* in einer bevorzugten Ausführungsform eine NADP⁺-abhängige Alkoholdehydrogenase. In einer bevorzugten Ausführungsform wird unter dem Begriff "Alkoholdehydrogenase", wie hierin verwendet, wird ein Enzym verstanden, das einen Aldehyd bzw. Keton zu dem entsprechenden primären bzw. sekundären oder sonstigem Alkohol oxidiert. Beispiele umfassend die NADP⁺-abhängigen Alkoholdehydrogenasen aus E. coli (YjgB, Datenbankcode ZP_07117674, und ein YahK, Datenbankcode BAE76108.1) und die Alkoholdehydrogenase aus *Ralstonia* sp. (SEQ ID NO: 5) sowie die jeweiligen Varianten davon. In einer besonders bevorzugten Ausführungsform handelt es sich um die Alkoholdehydrogenase aus *Ralstonia* sp. (SEQ ID NO: 5).

Erfindungsgemäß exprimiert die erfindungsgemäße Zelle neben der modifizierten Alanindehydrogenase aus *Bacillus subtilis* und/oder neben der NADP⁺-abhängigen Alkoholdehydrogenase in einer bevorzugten Ausführungsform eine Transaminase. In einer bevorzugten Ausführungsform wird unter dem Begriff "Transaminase", wie hierin verwendet, ein Enzym verstanden, das die Übertragung von Aminogruppen von einem Donor-, bevorzugt eine Aminosäure, auf ein Akzeptormolekül, bevorzugt eine Ketocarbonsäure, katalysiert. In einer besonders bevorzugten Ausführungsform ist die Transaminase aus der Gruppe ausgewählt, die die ω-Transaminase aus *Chromobacterium violaceum* (Datenbankcode NP_901695), *Pseudomonas putida* (Datenbankcode YP_001668026), *Pseudomonas putida* (Datenbankencode YP_001668026.1 or YP_001671460); *Rhodobacter sphaeroides* (strain ATCC 17023; Datenbankcode YP_353455) sowie Varianten davon umfasst, und es handelt sich bevorzugt um die Transaminase aus *Chromobacterium violaceum* (Datenbankcode NP_901695).

Im Falle der Verwendung wenigstens eines Ganzzellkatalysators ist bei längerer Reaktionszeit zu beachten, dass die Bedingungen mit der Lebensfähigkeit der wenigstens einen als Ganzzellkatalysator eingesetzten Zelle kompatibel sind. Der Fachmann kann Standardwerken, beispielsweise Fuchs/Schlegel (2007) Allgemeine Mikrobiologie, 2008, Georg Thieme Verlag, Bedingungen und Lösungen entnehmen, die die Erhaltung solcher Zellen in einem lebensfähigen Zustand ermöglichen.

Das erfindungsgemäße Verfahren kann auf eine große Anzahl von industriell relevanten Alkoholen angewandt werden. In einer bevorzugten Ausführungsform handelt es sich dabei um eine ω-Hydroxycarbonsäure oder einen Ester, bevorzugt Methylester, davon, die oder der zu einer ω-Aminocarbonsäure oxidiert und aminiert wird. In einer weiteren Ausführungsform handelt es sich um ein Diol, das zu einem Diamin oxidiert und aminiert wird. In einer weiteren bevorzugten Ausführungsform handelt es sich bei dem primären Alkohol um ein Hydroxyalkylamin. Die Länge der Kohlenstoffkette ist dabei variabel und x beträgt wenigstens 3. Bevorzugt weist die Kohlenstoffkette mehr als drei C-Atome auf, d. h. x = 4, 5, 6, 7, 8, 9, 10, 11, 12, 13, 14, 15, 16, 17, 18, 19, 20 oder mehr. Beispielhafte Verbindungen umfassen ω-Hydroxy-Laurinsäure, ω-Hydroxy-Laurinsäure-Methylester, und Alkan-Diole, insbesondere 1,8-OctanDiol und 1,10-Dekan-Diol.

In Frage kommen weiterhin α-Hydroxycarbonsäuren, bevorzugt diejenigen, die zu den α-Ketocarbonsäuren oxidiert werden können, d. h. solchen der Formel R^{s}-C(OH)H-COOH, die wiederum durch Aminierung zu den proteinogenen Aminosäuren umgewandelt werden können, darunter insbesondere essentielle Aminosäuren wie Methionin und Lysin. Konkrete Beispiele umfassend die Säuren, bei denen R^{s} ein Substituent aus der Gruppe umfassend, H, Methyl, - (CH₂)₄-NH₂, -(CH₂)₃-NH-NH-NH₂, -CH₂-CH₂-S-CH₃, -CH(CH₃)₂, -CH₂-CH(CH₃)₂, -CH₂-(1H-indol-3-yl), -CH(OH)-CH₃, -CH₂-Phenyl, -CH(CH₃)-CH₂-CH₃ ist. Weitere sekundäre Alkohole umfassen 2-Alkanole, z. B. 2-Propanol, 2-Butanol, 2-Pentanol, 2-Hexanol usw. Weiter kommen sekundäre mehrwertige Alkohole in Betracht, beispielsweise Alkandiole wie Ethandiol, Alkantriole wie Glycerin und Pentaerythrit in Betracht. Weitere Beispiele umfassen Cycloalkanole, bevorzugt Cyclohexanol und Bis(p-hydroxycyclohexyl)methan, die Alkohole der Gruppe H₃C - C(OH)H - (CH₂)ₓ - R⁴, wobei R⁴ aus der Gruppe ausgewählt ist, die -OH, -SH, - NH₂ und -COOR⁵ umfasst, x wenigstens 3 ist und R⁵ aus der Gruppe ausgewählt ist, die H, Alkyl und Aryl umfasst.

Die Länge der Kohlenstoffkette ist im Falle von Alkoholen der Formel Alkohole der H₃C - C(OH)H - (CH₂)ₓ - R⁴ variabel und x beträgt wenigstens 3. Bevorzugt weist die Kohlenstoffkette mehr als drei C-Atome auf, d. h. x = 4, 5, 6, 7, 8, 9, 10, 11, 12, 13, 14, 15, 16, 17, 18, 19, 20 oder mehr. Zahlreiche sekundäre Alkohole sind kommerziell erhältlich und können in käuflicher Form direkt eingesetzt werden. Alternativ kann der sekundäre Alkohol zuvor oder *in situ* biotechnologisch erzeugt werden, beispielsweise durch Hydroxylierung eines Alkans durch geeignete Alkanoxidasen, bevorzugt Monooxygenasen, erzeugt werden.

In einer besonders bevorzugten Ausführungsform ist R⁴ im Falle von sekundären Alkoholen der Formel H₃C - C(OH)H - (CH₂)ₓ - R⁴ aus der Gruppe ausgewählt, die -OH und -COOR⁵ umfasst, x ist wenigstens 11 und R⁵ ist aus der Gruppe ausgewählt, die H, Methyl, Ethyl und Propyl umfasst.

Besonders bevorzugt handelt es sich bei dem primären oder sekundären Alkohol um einen Zuckeralkohol, worunter in einer bevorzugten Ausführungsform, wie hierin, verwendet, ein Kohlenhydrat mit wenigstens einer Hydroxygruppe verstanden wird. In einer besonders bevorzugten Ausführungsform handelt es sich um einen dizyklischen Zuckeralkohol. Unter einem "dizyklischen Zuckeralkohol" wird in einer bevorzugten Ausführungsform, wie hierin verstanden, ein Zuckeralkohol verstanden, der wenigstens transient, zwei Ringsysteme ausbilden kann. In einer bevorzugtesten Ausführungsform handelt es sich bei einem Zuckeralkohol um ein Dianhydrohexitol oder eine Verbindung aus der Gruppe umfassend 1,4:3,6-Dianhydro-D-Mannitol, 1,4:3,6-Dianhydro-D-Glucitol (Isosorbid) und 1,4:3,6-Dianhydro-L-Iditol.

Stehen die erfindungsgemäß umzusetzenden Alkohole nicht also solche, sondern nur in Form von Alkanvorläufern zur Verfügung, so besteht die Möglichkeit, dass die erfindungsgemäße Zelle zusätzlich eine Monooxygenase exprimiert oder das erfindungsgemäße Verfahren um einen Schritt erweitert wird, in dem eine Monooxygenase ein Alkan hydroxyliert. In einer besonders bevorzugten Ausführungsform handelt es sich bei der Monooxygenase um eine Monooxygenase aus der AlkB-Familie. AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig. AlkT wird als FAD-abhängige Rubredoxin-Reduktase charakterisiert, die Elektronen von NADH auf AlkG überträgt. AlkG ist ein Rubredoxin, ein eisenhaltiges Redoxprotein, das als direkter Elektronendonor für AlkB fungiert. In einer bevorzugten Ausführungsform wird unter dem Begriff "Monooxygenase aus der alkB-Familie", wie hierein verwendet, eine membranständige Alkanhydroxylase verstanden. In einer weiteren bevorzugten Ausführungsform wird unter dem selben Begriff "Alkanhydroxylase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1, dieser Datenbankcode und sämtliche weitere in diesem Dokument verwendete Datenbankcodes stammen aus der Genbank Protein-Datenbank des NCBI in dem am 9. November 2011 verfügbaren Release) verstanden. Der Begriff "Sequenz", wie hierin verwendet, kann sich auf die Aminosäuresequenz eines Polypeptides und/oder die dafür codierende Nukleinsäuresequenz beziehen.

In einer weiteren bevorzugten Ausführungsform handelt es sich bei der Monooxygenase um Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. In einer bevorzugten Ausführungsform ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens fünf, bevorzugt zwölf Kohlenstoffstoffreste zu katalysieren. In einer weiteren bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante davon, die bevorzugt Alkanhydroxylaseaktivität aufweist.

Die vorliegenden Anmeldung umfasst ein Sequenzprotokoll mit den folgenden Polypeptid (Polyp)- und Nukleotid (DNA)-Sequenzen:

| SEQ ID NO | Typ | Beschreibung |
|---|---|---|
| 1 | Polyp | Alanin-Dehydrogenase von *Bacillus subtilis* mit Leu197Arg und Asp196Ala |
| 2 | DNA | Nukleotidsequenz kodierend für Alanin-Dehydrogenase von *Bacillus subtilis* mit Leu197Arg und Asp196Ala |
| 3 | Polyp | Alkoholdehydrogenasen aus E. *coli* (YjgB, Datenbankcode ZP_07117674,) |
| 4 | Polyp | Alkoholdehydrogenasen aus E. *coli* (YahK, Datenbankcode BAE76108.1) |
| 5 | Polyp | Alkoholdehydrogenase aus *Ralstonia* sp. (zuvor SEQ ID 10) |
| 6 | Polyp | Transaminase aus *Chromobacterium violaceum* (Datenbankcode NP_901695) |
| 7 | Polyp | Aminotransferase aus *Pseudomonas putida* (Datenbankcode YP_001668026) |
| 8 | Polyp | Aminotransferase aus *Pseudomonas putida* (Datenbankencode YP_001668026.1) |
| 9 | Polyp | Aminotransferase aus *Pseudomonas putida* (Datenbankencode YP_001671460) |
| 10 | Polyp | Aminotransferase aus *Rhodobacter sphaeroides* (strain ATCC 17023; Datenbankcode YP_353455) |
| 11 | DNA | Oligodesoxynukleotid AlaDHfw (zuvor Seq ID 5) |
| 12 | DNA | Oligodesoxynukleotid AlaDHrv (zuvor 6) |
| 13 | DNA | Oligodesoxynukleotid AlaDH_D196A/L197Rfw (zuvor 8) |
| 14 | DNA | Oligodesoxynukleotid AlaDH_D196A/L197Rrv (zuvor 9) |
| 15 | DNA | Oligodesoxynukleotid ADHfw (zuvor 11) |
| 16 | DNA | Oligodesoxynukleotid ADHrv (zuvor 12) |
| 17 | Polyp | Aminotransferase aus *Paracoccus denitrificans (zuvor 14)* |
| 18 | DNA | Oligodesoxynukleotid pCR6fw (zuvor 15) |
| 19 | DNA | Oligodesoxynukleotid pCR6rv (zuvor 16) |
| 20 | DNA | Oligodesoxynukleotid pCR6_L417Mfw (zuvor 18) |
| 21 | DNA | Oligodesoxynukleotid pCR6_L417Mrv (zuvor 19) |
| 22 | Polyp | Alanin-Dehydrogenase von *Bacillus subtilis* mit Leu197Arg und Asp196Ala mit His-Tag |
| 23 | DNA | Nukleotidsequenz kodierend für Alanin-Dehydrogenase von *Bacillus subtilis* mit Leu197Arg und Asp196Ala mit His-Tag |

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

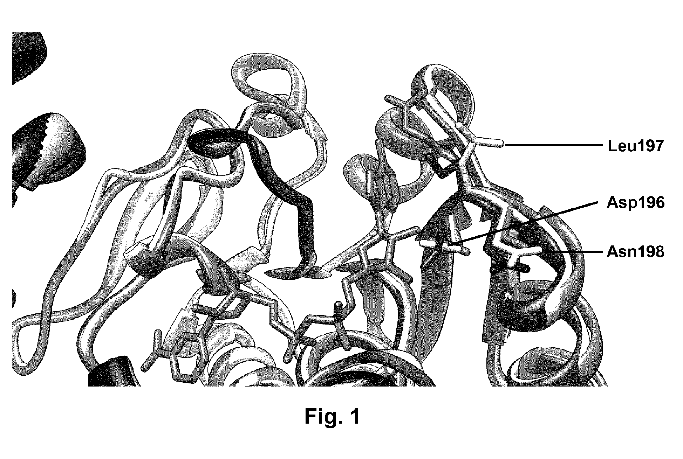

**Fig. 1** zeigt die strukturelle Überlagerung des Homologiemodells der AlaDH aus *Bacillus subtilis* (weiss; SEQ ID NO: 1), des Homologiemodells der AlaDH aus *Shewanella* sp. (grau) und der Kristallstruktur der AlaDH aus *Mycobacterium tuberculosis* (schwarz; PDB Code: 2VHW). Die dargestellte Aminosäure Asp196 der BasAlaDH entspricht der Aminosäure Asp198 in der SheAlaDH bzw. Aminosäure Asp198 in der MtAlaDH. Die Aminosäure Leu197 der BasAlaDH entspricht der Aminosäure Arg199 in der SheAlaDH bzw. Aminosäure Ile199 in der MtAlaDH. Die Aminosäure Asn198 der BasAlaDH entspricht der Aminosäure Ser200 in der SheAlaDH bzw. Aminosäure Asn200 der MtAlaDH.

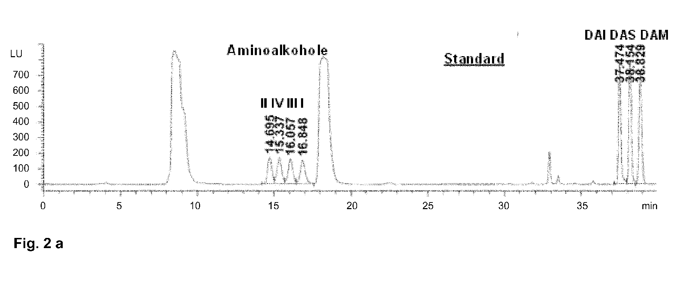

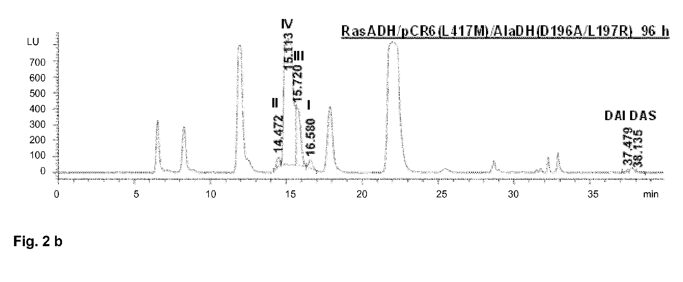

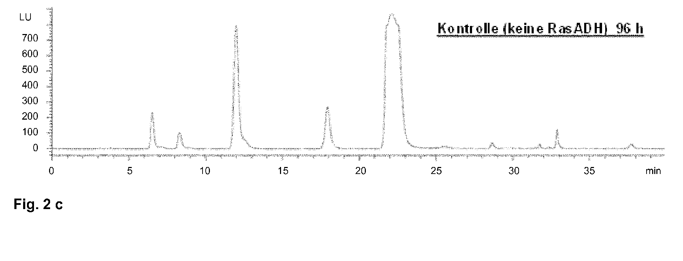

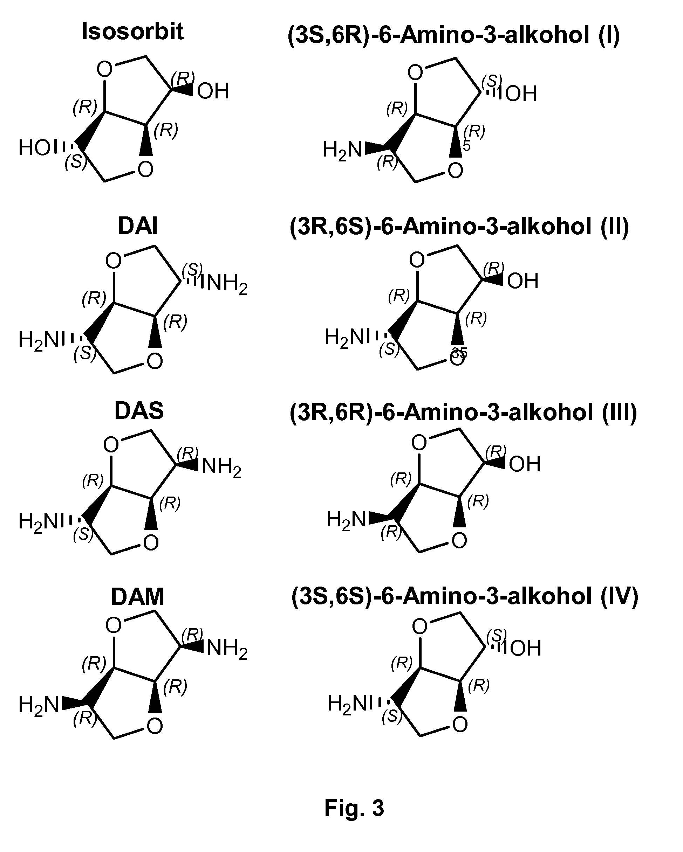

**Fig. 2** zeigt die FMOC/HPLC-Analyse der durch die drei Enzyme RasADH, pCR6(L417M) und AlaDH(D196A/L197R) katalysierten Umsetzung von Isosorbit und Ammoniumsalz nach 96 h. Gezeigt sind (a) die Standards (je 1 mM der Aminoalkohole I, II, III und IV gemäß **Fig. 3** + je 1 mM der Diamine DAI, DAS und DAM), (b) die durch RasADH, pCR6(L417M) und AlaDH(D196A/L197R) katalysierte Reaktion nach 96 h, (c) die Kontrollreaktion wie bei (b) aber ohne RasADH nach 96 h. Zur Derivatisierung wurden 20 µl der jeweiligen Reaktionsprobe in ein HPLC-Vial mit 60 µl 0,5 M Natriumborat pH 9,0 überführt, gut gemischt und 80 µl FMOC-Reagenz (Alltech Grom) hinzugegeben. Überschüssiges FMOC-Reagenz wurde durch Zugabe von 100 µl EVA-Reagenz (Alltech Grom) abgefangen. Durch Zufügen von 440 µl 50 mM Natriumacetat, pH 4,2 + 70 % Acetonitril (v/v) wurden die Bedingungen für die HPLC-Analyse eingestellt. Chromatographische Bedingungen: Agilent SB-C8 Säule (4,6 × 150 mm); Flussrate: 1ml/min; Injektionsvolumen: 20 µl; Puffer A. 50 mM NaAcetat pH 4,2 + 20 % Acetonitril (v/v); Puffer B: 50 mM NaAcetat pH 4,2 + 95 % Acetonitril (v/v); Gradient: 0 min 16 % B, 5 min 16 % B, 25 min 18 % B, 28 min 52 % B, 40 min 25 % B.
**Fig. 3** zeigt die chemischen Formeln des Ausgangssubstrats Isosorbit (1,4:3,6-Dianhydro-D-Sorbit(ol)), der Stereoisomere des Aminoalkohols (I bis IV) sowie der stereoisomeren Formen des Diamin-Endproduktes (DAI: 2,5-Diamino-1,4:3,6-Dianhydro-2,5-Didesoxy-L-Idit(ol), DAS: 2,5-Diamino-1,4:3,6-Dianhydro-2,5-Didesoxy-D-Sorbit(ol) und DAM: 2,5-Diamino-1,4:3,6-Dianhydro-2,5-Didesoxy-D-Mannit(ol).

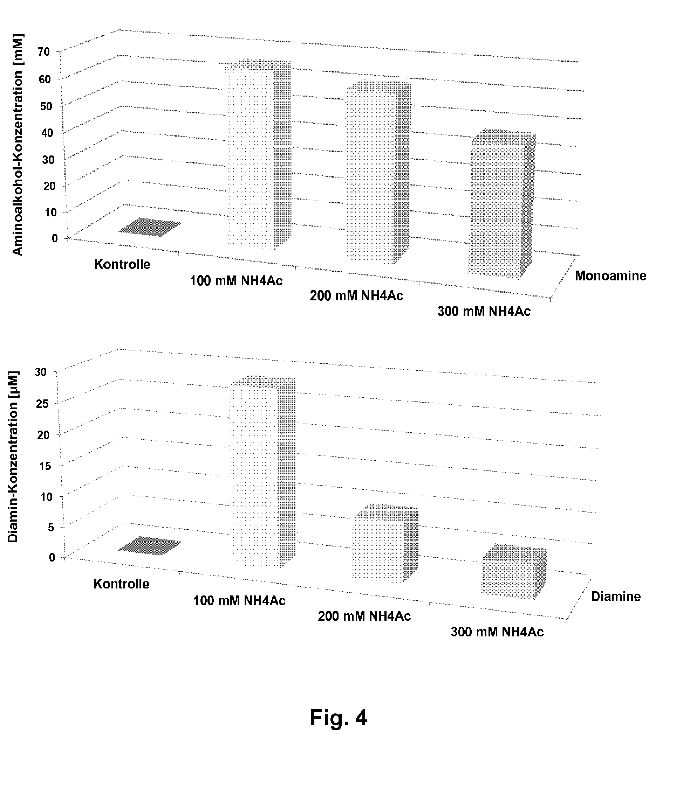

**Fig. 4** zeigt die Ausbeuten an Mono- und Diamin aus der FMOC/HPLC-Analyse der durch RasADH, pCR6(L417M) und AlaDH(D196A/L197R) katalysierten Umsetzung von Isosorbit und Ammoniumacetat bei unterschiedlichen Ammoniumkonzentrationen. Reaktionsbedingungen: 300 mM Isosorbit, 2 mM NADP⁺, 100 - 300 mM NH₄OAc, 5 mM L-Alanin, 0,3 mM PLP, 132 µM RasADH, 40 µM pCR6(L417M), 24 µM AlaDH(D196A/L197R) in 25 mM Hepes/NaOH, pH 8,3; Inkubation bei 30 °C.

### Beispiel 1: Änderung der Cosubstratspezifität einer Alanin-Dehydrogenase durch bioinformatische Analyse und Protein-Engineering

Strukturhomologa der L-Alanin-Dehydrogenase (AlaDH) aus *Bacillus subtilis* (BasAlaDH; SEQ ID NO: 1) wurden mit Hilfe des Servers HHpred (Bioinformatics Toolkit; MPI Tübingen) identifiziert. Dabei wurde ein globales multiples Sequenzalignment (MSA) mit HHblits (Bioinformatics Toolkit; MPI Tübingen; URL: http://toolkit.tuebingen.mpg.de/hhpred) in drei Iterationen und mit aktiviertem "Secondary Structure Score" generiert. Als ähnlichstes Protein (51 % Sequenzidentität) erwies sich die AlaDH aus *Mycobacterium tuberculosis* (MtAlaDH). Die Kristallstrukturen dieser AlaDH (PDB Codes: 2VHW, 2VHX, 2VHY und 2VHZ) wurden als Strukturvorlage zur Generierung von Homologiemodellen der BasAlaDH und der AlaDH aus *Shewanella* sp. (SheAlaDH) mittels des HHpred Servers verwendet (**Fig. 1**).

Die MtAlaDH besitzt wie das Enzym aus *Bacillus subtilis* eine Redoxfaktorspezifität für NADH, wohingegen die SheAlaDH als einzige bekannte AlaDH in der Lage ist, sowohl NADH als NADPH enzymatisch umzusetzen (Ashida et al. (2004) J. Mol. Catal. B: Enzym. 30, 173-176). Eine strukturelle Überlagerung der Homologiemodelle von BasAlaDH und SheAlaDH mit der Referenz-Struktur der MtAlaDH wurde mit dem Programm Chimera (Version 1.5.3; URL: http://www.cgl.ucsf.edu/chimera/ mit der Funktion "Matchmaker"; URL http://www.cgl.ucsf.edu/chimera/docs/ContributedSoftware/matchmaker/matchmaker.html) berechnet (**Fig. 1**). Dazu wurde der Needleman-Wunsch Algorithmus unter Verwendung der Matrize Blossum-62 benutzt und der "Secondary Structure Score" auf 30 % gesetzt. Als Iterationsabbruch diente eine Distanz von 1.0 Å zwischen paarweisen Atomen.

Die strukturelle Überlagerung der genannten drei AlaDHs zeigt in der Nähe des "Rossmann Fold"-Motivs einen für die NADH-Spezifität offenbar verantwortlichen konservierten Aspartat-Rest (Asp196 in SEQ ID NO: 1; siehe **Fig. 1**)

Der konservierte Aspartat-Rest war Ausgangspunkt für die Konstruktion der BasAlaDH Enzymvarianten D196A/L197R, wobei die Struktur der SheAlaDH als Template verwendet wurde. Wie die strukturelle Überlagerung zeigt (**Fig. 1**), wird durch den Austausch D196A eine Ausbildung von Wasserstoffbrücken von Asp196 zum 2'- und 3'-OH der Ribose des NADH verhindert, aber die Bindung des am 2'-OH der Ribose befindlichen Phosphats des NADPH an das Enzym ermöglicht. Durch die Substitution L197R wird die neue Cosubstratspezifität in Folge der Ausbildung von Salzbrücken zwischen dem eingeführten Arginin-Rest und der Phosphat-Gruppe des NADPH weiter begünstigt.

Das Gen der AlaDH aus *Bacillus subtilis* (SEQ ID NO: 4) wurde durch Polymerase-Kettenreaktion (PCR) unter Verwendung der Oligodesoxynukleotide AlaDHfw (SEQ ID NO: 11) und AlaDHrv (SEQ ID NO: 12) von dem Plasmid pUC18-AlaDH amplifiziert, mit dem Restriktionsenzym Kpnl am 3'-Ende geschnitten und mit dem Expressionsvektor pASK-IBA35(+) (IBA GmbH, Göttingen), der mit den Restriktionsenzymen Ehel und Kpnl geschnitten wurde, ligiert. Das resultierende Expressionsplasmid pASK-IBA35(+)-AlaDH, auf dem die BasAlaDH mit einem N-terminalem His₆-tag kodiert ist, wurde mittels analytischem Restriktionsverdau sowie DNA-Sequenzierung verifiziert. Das für die Enzymvariante D196A/L197R kodierende Plasmid wurde durch ortsgerichtete Mutagenese nach der QuikChange-Methode unter Verwendung der Oligodesoxynukleotide AlaDH_D196A/L197Rfw (SEQ ID NO: 13) und AlaDH_D196A/L197Rrv (SEQ ID NO: 14) erzeugt. Das resultierende Expressionsplasmid pASK-IBA35(+)-AlaDH(D196A/L197R) wurde mittels DNA-Sequenzierung verifiziert.

Die Expressionsplasmide für die BasAlaDH sowie deren Variante D196A/L197R wurden anschließend zur Transformation von E. *coli* BL21 verwendet (Sambrook et al. (2001) Molecular cloning: a laboratory manual, 3rd edition. Cold Spring Harbor Laboratory Press). Die Genexpression wurde jeweils in LB-Medium (Sambrook et al. 2001) mit 100 µg/ml Ampicillin (2 L Kulturvolumen im 5 L-Schüttelkolben) bei 30 °C in der exponentiellen Wachstumsphase bei OD₅₅₀ = 0,5 durch Zugabe von 0,2 µg/ml Anhydrotetracyclin (aTc; Acros, Geel, Belgien) induziert. Nach einer Induktionszeit von 3 h wurde die Kultur geerntet und die Zellen in 40 mM Hepes/NaOH pH 7,5, 0,5 M NaCl aufgenommen und in einem French-Press Homogenisator (G. Heinemann, Schwäbisch Gmünd) mechanisch aufgeschlossen. Der klare Überstand wurde auf eine mit Zn²⁺ beladene Chelating Sepharose™ Fast Flow Säule (2.8 mL Bettvolumen; GE Healthcare, München) aufgetragen und die mit dem His₆-tag fusionierten Enzyme mit einem linearen Imidazol/HCl-Konzentrationsgradienten von 0 bis 500 mM in 40 mM Hepes/NaOH pH 7,5, 0,5 M NaCl eluiert. Die Elutionsfraktionen wurden durch Ultrafiltration konzentriert und mittels Gelfiltration an Superdex200 (GE Healthcare) in Gegenwart von 25 mM Hepes/NaOH pH 8,3 chromatographisch gereinigt.

Die Michaelis-Konstante (K_{M}) und die Wechselzahl (k_{cat}) der beiden gereinigten Enzyme wurden für die Cosubstrate NADH sowie NADPH anhand der reduktiven Aminierung von Pyruvat bestimmt und die katalytische Effizienz berechnet (k_{cat}/K_{M}) (Tabelle 1). Die Enzymtests setzten sich wie folgt zusammen:

| **Reagenz bzw. Enzym** | **Endkonzentration im Ansatz** |
|---|---|
| Pyruvat | 5 mM |
| Ammoniumacetat | 200 mM |
| NADH/H⁺ bzw. NADPH/H⁺ | 10 - 500 µM |
| BasAlaDH (Wildtyp bzw. Doppelmutante D196A/L197R) | 0.5 nM |
| Hepes/NaOH-Puffer pH 8,3 | 25 mM |
| Gesamtvolumen | 250 µl |

Dabei wurde gefunden, dass durch die Substitutionen D196A und L197R die Cosubstratspezifität der BasAlaDH erfolgreich von NADH auf NADPH geändert wurde, wobei die katalytische Effizienz hinsichtlich der reduktiven Aminierung von Pyruvat nahezu erhalten blieb.

**Tabelle 1: K_{M}- und k_{cat}-Werte für die durch BasAlaDH katalysierte reduktive Aminierung von Pyruvat mit NADH bzw. NADPH.**

| **Enzym** | **NADH K_{M}** [µM] ¹/min⁻¹] | **k_{cat}** [min⁻¹] | **k_{cat}/K_{M}** [µM⁻ | **NADPH K_{M}** [µM] ¹/min⁻¹] | **k_{cat}** [min⁻¹] | **k_{cat}/K_{M}** [µM⁻ |
|---|---|---|---|---|---|---|
| Wildtyp AlaDH | 14±2 | 838 ± 21 | 59.9 | 448 ± 135 | 666 ± 114 | 1.5 |
| Mutante D196A/L197R | 887 ± 360 | 2669 ± 719 | 3.0 | 32 ± 3 | 1730 ± 39 | 54.1 |

### Beispiel 2: Synthese von Mono- und Diaminen aus Isosorbit und Ammoniumsalzen durch gekoppelte enzymatische Reaktion einer Alkoholdehydrogenase, einer Aminotransferase und einer Alanin-Dehydrogenase

Das Strukturgen der Alkoholdehydrogenase aus *Ralstonia* sp. (SEQ ID NO: 5) wurde durch PCR unter Verwendung der Oligodesoxynukleotide ADHfw (SEQ ID NO: 15) und ADHrv (SEQ ID NO: 16) von dem Plasmid pEam-RasADH (Lavandera et al. (2008) J. Org. Chem. 73, 6003-6005) amplifiziert, mit dem Restriktionsenzym *Kpnl* am 3'-Ende geschnitten und schließlich mit dem Expressionsvektor pASK-IBA35(+), der mit den Restriktionsenzymen Ehel und *Kpnl* geschnitten wurde, ligiert. Das resultierende Expressionsplasmid pASK-IBA35(+)-RasADH, auf dem die Alkoholdehydrogenase mit einem N-terminalem His₆-tag kodiert ist, wurde durch analytischen Restriktionsverdau sowie DNA-Sequenzierung verifiziert.

Das Gen der Aminotransferase aus *Paracoccus denitrificans* (SEQ ID NO: 17) wurde durch PCR unter Verwendung der Oligodesoxynukleotide pCR6fw (SEQ ID NO: 18) und pCR6rv (SEQ ID NO: 19) von dem Plasmid pET21a(+)-pCR6 amplifiziert, mit dem Restriktionsenzym *Hind*III am 3'-Ende geschnitten und schließlich mit dem Expressionsvektor pASK-IBA35(+), der mit den Restriktionsenzymen *Eh*el und *Hin*dIII geschnitten wurde, ligiert. Das resultierende Expressionsplasmid pASK-IBA35(+)-pCR6, auf dem die Aminotransferase mit einem N-terminalem His₆-tag kodiert ist, wurde durch analytischen Restriktionsverdau sowie DNA-Sequenzierung verifiziert. Das für die Enzymvariante L417M der Aminotransferase codierende Plasmid wurde mittels ortsgerichteter Mutagenese des Plasmids pASK-IBA35(+)-pCR6 nach der QuikChange-Methode (Agilent, Waldbronn) unter Verwendung der Oligodesoxynukleotide pCR6_L417Mfw (SEQ ID NO: 20) und pCR6_L417Mrv (SEQ ID NO: 21) erzeugt. Das resultierende Expressionsplasmid pASK-IBA35(+)-pCR6(L417M) wurde mittels DNA-Sequenzierung verifiziert.

Als Expressionsplasmid für die D196A/L197R-Mutante der AlaDH aus *Bacillus subtilis* (SEQ ID NO: 1) wurde pASK-IBA35(+)-AlaDH(D196A/L197R) aus Beispiel 1 verwendet.

Die Expressionsplasmide pASK-IBA35(+)-RasADH, pASK-IBA35(+)-pCR6(L417M) und pASK-IBA35(+)-AlaDH(D196A/L197R) für die drei Enzyme wurden anschließend jeweils zur Transformation von *E. coli* BL21 verwendet. Die Genexpression in den drei resultierenden Stämmen wurde jeweils in LB-Medium mit 100 □g/ml Ampicillin (2 L Kulturvolumen im 5 L-Schüttelkolben) bei 30 °C in der exponentiellen Wachstumsphase bei OD₅₅₀ = 0,5 durch Zugabe von 0,2 µg/ml aTc induziert. Nach einer Induktionszeit von 3 h wurde die Kultur geerntet und die Zellen in 40 mM Hepes/NaOH pH 7,5, 0,5 M NaCl aufgenommen und in einem French-Press Homogenisator mechanisch aufgeschlossen. Der klare Überstand wurde auf eine mit Zn²⁺ beladene Chelating Sepharose™ Fast Flow Säule aufgetragen und die mit dem His₆-tag fusionierten Enzyme mit einem linearen Imidazol/HCl-Konzentrationsgradienten von 0 bis 500 mM in 40 mM Hepes/NaOH pH 7,5, 0,5 M NaCl eluiert. Die Elutionsfraktionen wurden durch Ultrafiltration konzentriert und mittels Gelfiltration an Superdex200 in Gegenwart von 25 mM Hepes/NaOH pH 8,3 chromatographisch gereinigt.

Die drei gereinigten Enzyme wurden direkt für die Aminierung des Isosorbits (1,4:3,6-Dianhydro-D-Sorbit(ol)) unter Recycling der Redoxfaktoren NADP⁺ und L-Alanin eingesetzt. Der Enzymtest setzte sich wie folgt zusammen:

| **Reagenz bzw. Enzym** | **Endkonzentration im Ansatz** |
|---|---|
| Hepes/NaOH-Puffer pH 8,3 | 25 mM |
| Isosorbit | 300 mM |
| NADP⁺ | 2 mM |
| L-Alanin | 5 mM |
| Pyridoxalphosphat (PLP) | 0,3 mM |
| Ammoniumacetat (NH₄OAc) | 100 - 300 mM |
| Alkoholdehydrogenase | 132 µM |
| Aminotransferase (L417M) | 40 µM |
| Alanin-Dehydrogenase (D196A/L197R) | 24 µM |
| Gesamtvolumen | 250 µl |

Nach Inkubation für einen Zeitraum von 0 bis 96 h bei 30 °C wurde die Bildung von Mono- und Diaminen als Reaktionsprodukte unter Zugabe eines Überschusses an FMOC-Reagenz (Alltech Grom, Rottenburg-Hailfingen; siehe **Fig. 1**) durch HPLC (Agilent 1200 Serie; siehe **Fig. 2**) mit einem Fluoreszenz-Detektors nachgewiesen und quantifiziert.

**Fig. 2** (Mitte) zeigt das Chromatogramm der Auftrennung des Reaktionsgemisches. Bei den Retentionszeiten von 15,113 min, 15,720 min, 16,580 und 14,472 min wurden Produkt-Peaks beobachtet, die anhand der mitgeführten Standards dem 6-Amino-3-alkohol in den 4 möglichen stereoisomeren Formen gemäß **Fig. 3** (IV: 3S,6S; III: 3R,6R; I: 3S,6R; II: 3R,6S) zugeordnet werden konnten. Diese Peaks traten bei der FMOC/HPLC-Analyse in der Negativkontrolle (analog angesetzter und inkubierter Reaktionsansatz, aber ohne Alkoholdehydrogenase) nicht auf. Darüber hinaus wurden nach einer Reaktionszeit von 96 h weitere Produkt-Peaks bei Retentionszeiten von 37,479 min und 38,135 min erhalten. Diese lassen sich anhand der Standards den Diaminen 2,5-Diamino-1,4:3,6-Dianhydro-2,5-Didesoxy-L-Idit(ol) (DAI) und 2,5-Diamino-1,4:3,6-Dianhydro-2,5-Didesoxy-D-Sorbit(ol) (DAS) zuordnen.

Die Kopplung der drei enzymatischen Reaktionen ermöglicht also einen Nettoumsatz von Isosorbit zum (3S,6S)-6-Amino-3-alkohol als Hauptprodukt mit Recycling-Faktoren von 30 für NADP⁺ und 12 für L-Alanin (**Fig. 4**).

## Patentansprüche

1. Polypeptid umfassend die Aminosäuresequenz der Alanindehydrogenase aus *Bacillus subtilis* oder eine Variante davon, wobei Leu197 oder eine an homologer Position in der Aminosäuresequenz befindliche Aminosäure gegen eine Aminosäure mit positiv geladener Seitenkette ausgetauscht ist.

2. Polypeptid nach Anspruch 1, wobei es sich bei der Aminosäure mit positiv geladener Seitenkette um Arginin handelt.

3. Polypeptid nach einem der Ansprüche 1 bis 2, wobei zusätzlich Asp196 oder eine an homologer Position in der Aminosäuresequenz befindliche Aminosäure gegen eine Aminosäure mit neutraler oder positiv geladener Seitenkette ausgetauscht ist.

4. Polypeptid nach einem der Ansprüche 1 bis 3, wobei es sich bei der Aminosäure mit neutraler oder positiv geladener Seitenkette um eine Aminosäure aus der Gruppe handelt, die Alanin, Glycin, Serin und Cystein umfasst, bevorzugt um Alanin.

5. Polypeptid nach Anspruch 4, wobei es sich um das in SEQ ID NO 1 dargestellte Polypeptid handelt.

6. Nukleinsäuremolekül umfassend eine für das Polypeptid nach einem der Ansprüche 1 bis 4 kodierende Nukleotidsequenz.

7. Nukleinsäuremolekül nach Anspruch 6, wobei es sich um die in SEQ ID NO 2 dargestellte Nukleotidsequenz handelt.

8. Vektor umfassend das Nukleinsäuremolekül nach einem der Ansprüche 6 bis 7.

9. Zelle umfassend das Polypeptid nach einem der Ansprüche 1 bis 5, das Nukleinsäuremolekül nach einem der Ansprüche 6 bis 7 oder den Vektor nach Anspruch 8.

10. Zelle nach Anspruch 9, weiter exprimierend eine NADP⁺-abhängige Alkoholdehydrogenase.

11. Zelle nach einem der Ansprüche 9 oder 10, weiter exprimierend eine Transaminase.

12. Verfahren zur Herstellung von Alanin oder einer unter Verbrauch von Alanin entstehenden Verbindung, umfassend den Schritt
c) Umsetzen von Pyruvat mit Ammonium und NADPH zu Alanin durch Kontaktieren mit dem Polypeptid nach einem der Ansprüche 1 bis 5 oder mit der Zelle nach einem der Ansprüche 9 bis 11.

13. Verfahren nach Anspruch 10, weiter umfassend
a) Umsetzen eines primären bzw. sekundären Alkohols zum Aldehyd bzw. Keton durch Kontaktieren mit einer NADP⁺-abhängigen Alkoholdehydrogenase in Anwesenheit von NADP⁺.

14. Verfahren nach einem der Ansprüche 12 bis 13, weiter umfassend
b) Umsetzen des in Schritt a) hergestellten Aldehydes bzw. Ketons zum Amin durch Kontaktieren mit einer Transaminase in Anwesenheit von Alanin.

15. Verfahren nach einem der Ansprüche 12 bis 14, wobei die Schritte a), b) und c) in der gleichen Reaktionsmischung ablaufen, bevorzugt gleichzeitig.

16. Zelle oder Verfahren nach einem der Ansprüche 9 bis 15, wobei die Zelle sämtliche Enzyme aus der Gruppe umfassend das Polypeptid nach einem der Ansprüche 1 bis 5, die NADP⁺-abhängige Alkoholdehydrogenase und die Transaminase intrazellulär lokalisiert exprimiert.

17. Zelle oder Verfahren nach einem der Ansprüche 10 bis 16, wobei es sich bei der NADP⁺-abhängigen Alkoholdehydrogenase um eine NADP⁺-abhängige Alkoholdehydrogenase aus der Gruppe umfassend die Alkoholdehydrogenasen aus *E. coli* (YjgB, Datenbankcode ZP_07117674, und ein YahK, Datenbankcode BAE76108.1) und die Alkoholdehydrogenase aus *Ralstonia* sp. (SEQ ID NO: 10), bevorzugt um die Alkoholdehydrogenase aus *Ralstonia* sp. (SEQ ID NO: 10) oder Varianten davon handelt.

18. Zelle oder Verfahren nach einem der Ansprüche 11 bis 17, wobei es sich bei der Transaminase um eine Transaminase aus der Gruppe umfassend die Transaminasen aus *Chromobacterium violaceum* (Datenbankcode NP_901695), *Pseudomonas putida* (Datenbankcode YP_001668026), *Pseudomonas putida* (Datenbankencode YP_001668026.1 or YP_001671460); *Rhodobacter sphaeroides* (strain ATCC 17023; Datenbankcode YP_353455) sowie Varianten davon handelt, bevorzugt um die Transaminase aus *Chromobacterium violaceum* (Datenbankcode NP_901695).
